# EUROPEAN PATENT SPECIFICATION

(11) **EP 0 450 430 B1**
(45) Date of publication and mention of the grant of the patent: **25.06.1997**
(21) Application number: 91104558.1
(22) Date of filing: 22.03.1991
(51) Int. Cl.: C12N 15/53, C12N 9/02, C12P 21/02, C12N 1/14, C12P 7/10

(54) **DNA sequence comprising a structural gene coding for xylose reductase or xylose reductase and xylitol dehydrogenase**
DNS Sequenz, bestehend aus einem kodierenden, strukturellen Gen für Xylose-Reduktase oder Xylose -Reduktase und Xylitol-Dehydrogenase
Séquence d'ADN comprenant un gène codant pour la réductase de xylose ou la réductase de xylose et la déhydrogénase de xylitol

(30) Priority: 26.03.1990 DE 4009676
(43) Date of publication of application: 09.10.1991
(73) Proprietor: Rhein Biotech Gesellschaft für neue biotechnologische Prozesse und Produkte mbH, 40595 Düsseldorf (DE)
(72) Inventor: Strasser, Alexander W.M., Dr., W-4000 Düsseldorf 1 (DE); Hollenberg Cornelis P., Prof. Dr., W-4000 Düsseldorf (DE); Ciriacy-Wantrup, Michael von, Prof. Dr., W-4000 Düsseldorf 13 (DE); Kötter, Peter, W-4000 Düsseldorf 1 (DE); Amore, Rene, W-4000 Düsseldorf (DE); Piontek, Michael, W-4300 Essen 15 (DE); Hagedorn, Jutta, W-4000 Düsseldorf (DE)
(74) Representative: Grünecker, Kinkeldey, Stockmair & Schwanhäusser Anwaltssozietät

(56) References cited:
- EP-A- 0 238 023
- GB-A- 2 151 635
- US-A- 4 840 903
- Derwent Biotechnology Abstracts, Accession no. 86-08636 & Abstr., Annul.Meet.Am.Soc., 1986, Abstract 0-7; BOLEN et al:"Identification of a cDNA clone encoding aldose reductase from xylose-fermenting yeast Pachysolen tannophilus"
- CURRENT GENETICS, vol. 16, 1989, BERLIN, DE, pages 27-33; J. Hagedorn et al.: "Isolation and characterization of xyl mutants in a xylose-utilizing yeast, Pichia stipitis"
- Derwent Publications Ltd, London, GB, Database WPIL, accession no. 86-123031, week 8619 & JP61063291-DAIICHI KOGYO SEIYAKU, 01.04.86
- APPLIED MICROBIOLOGY AND BIOTECHNOLOGY, vol. 30, no.4, April 1989, BERLIN, DE, pages 351-357; R. Amore et al.: "The fermentation of xylose - an analysis of the expression of Bacillus and Actinoplanes xylose isomerase genes in yeast"
- Derwent Publication Limited, London,GB Database WPIL, accession no. 85-287878, Week 8546 & JP60199383-Morito S.-, 08.10.81
- Current Genetics, vol. 18, 1990, Berlin, DE; P. KÖTTER et al, pages 493-500

## Description

The present invention relates to a DNA sequence, a combination of DNA sequences, a vector, a microorganism, a method for producing xylose reductase or xylose reductase and xylitol dehydrogenase; the invention further relates to an ethanol manufacturing process, a process for production of biomass, a process for recycling of NADP⁺ from NADPH and a method for producing a desired protein in Pichia stipitis.

D-xylose is one of the most abundant carbohydrates occurring in plant biomass and wood. In the process of cellulose production, it is formed as a waste product from hydrolysis of xylan, which is the major compound of hemicellulose. To optimize the use of renewable carbon sources, it is desirable to convert xylose into ethanol or biomass. There are several yeast species, such as Candida (Gong et al., 1981, Jeffries, 1983), Debaryomyces, Hansenula, Kluyveromyces, Metschnikowia, Pachysolen, Paecilomyces (Wu et al., 1986) and Pichia (Maleszka and Schneider 1982), which are able to utilize pentoses, including D-xylose, and D-ribose, however, only aerobically.

In general, pentoses utilized by yeasts (e.g. Pichia stipitis) must be isomerized to pentuloses in order to be phosphorylated. This isomerization occurs via a NAD(P)H linked reduction (reductase) to pentitols followed by NAD⁺-linked oxidation (dehydrogenase) of the pentitols to the corresponding D-pentuloses (Barnett, 1976). The yeast mainly used in bioethanol production, S. cerevisiae, can utilize xylulose, however, this yeast is not able to ferment pentoses (Jeffries, 1988). It cannot be excluded, that S. cerevisiae also contains genes, coding for pentose fermenting proteins which however are not expressed.

Pentose fermentation by S. cerevisiae may be possible by providing a xylose utilising pathway from a xylose metabolizing organism. However, although many attempts have been undertaken to express bacterial xylose isomerase genes in S. cerevisiae, no xylose fermentation could be obtained probably due to inefficient expression of the foreign gene (Sarthy et al., 1987, Amore et al., 1989, Chan et al., 1986 & 1989).

Therefore it is a primary object of the present invention to provide genes of the enzymes involved in xylose degradation in order to be able to manipulate these genes, for example to combine these sequences with suitable regulating sequences.

This object has been solved by a DNA sequence comprising a structural gene coding for xylose reductase or xylose reductase and xylitol dehydrogenase and being capable of expressing said polypeptide(s) in a microorganism.

Further objects of the present invention will become apparent by the following detailed description of the invention, the examples and figures.

Throughout this application various publications are referenced by the first author within parenthesis.

Full citations of these references may be found at the end of the specification as an annex. The disclosures of these publications in their entireties are hereby incorporated by reference into this application in order to more fully describe the state of the art as known to those skilled therein as of the date of the invention described and claimed herein.

The DNA sequences according to the present invention preferably are derived from a yeast. Preferred yeast strains are selected from the genera Schwanniomyces, Saccharomyces, Kluyveromyces, Pichia, Hansenula, Candida, Debaryomyces, Metschnikowia, Pachysolen and Paecilomyces. All of these yeast genera are known to be able to convert xylose into ethanol using xylose reductase and xylitol dehydrogenase.

A preferred genus used as a source for the DNA sequence according to the present invention is the yeast Pichia. This genus comprises several species, any of which could be applied for performing the present invention. However, the preferred species is Pichia stipitis. The present inventors used Pichia stipitis CBS5773 for isolation of the DNA sequences comprising a structural gene coding for xylose reductase and/or xylitol dehydrogenase. Pichia stipitis CBS5773 was redeposited under the Budapest Treaty on March 21, 1990 (DSM 5855).

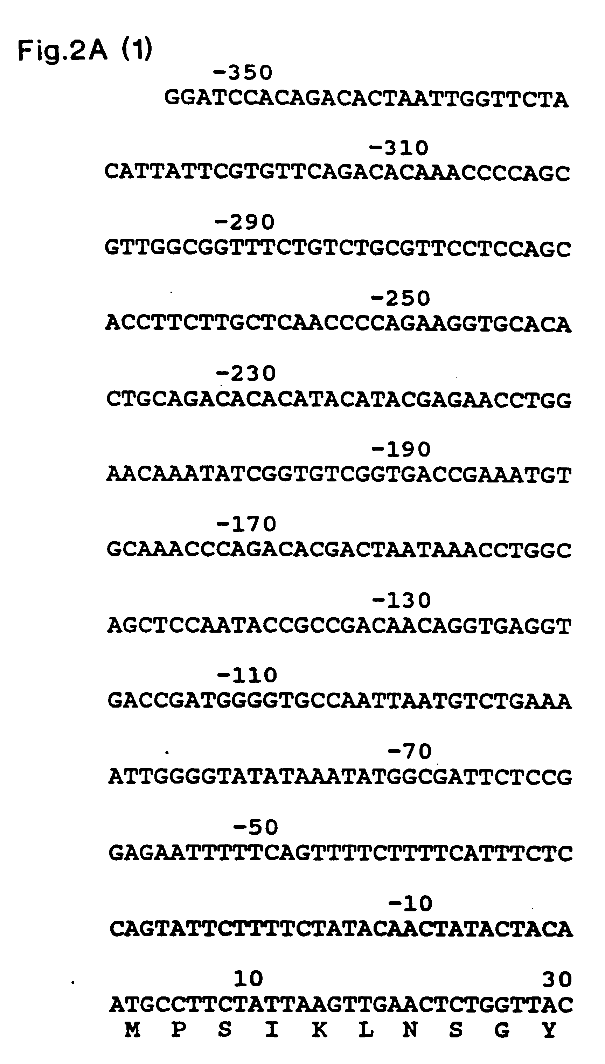

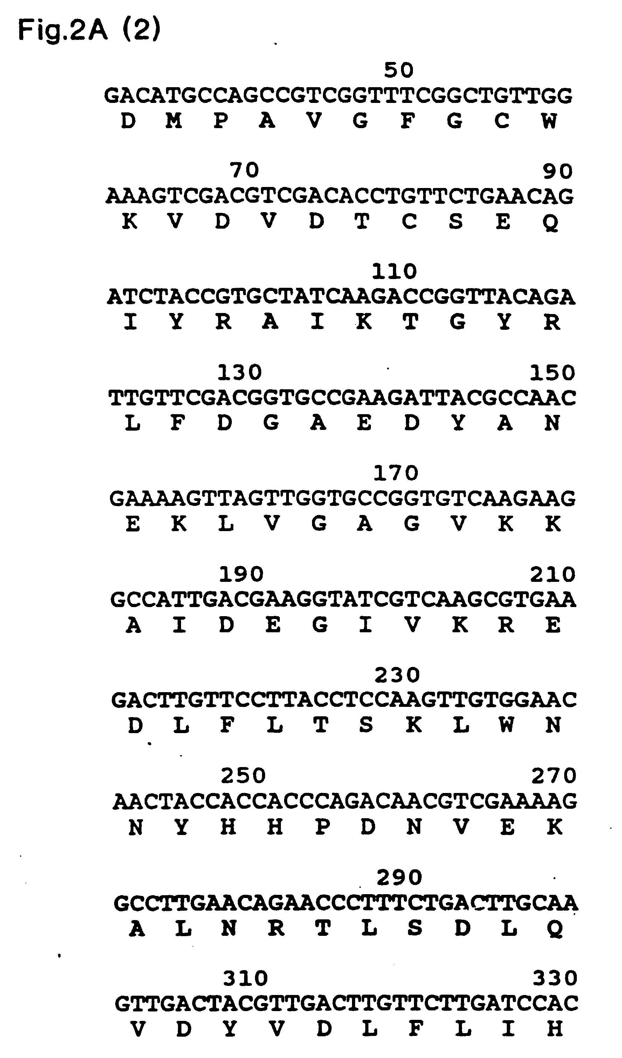

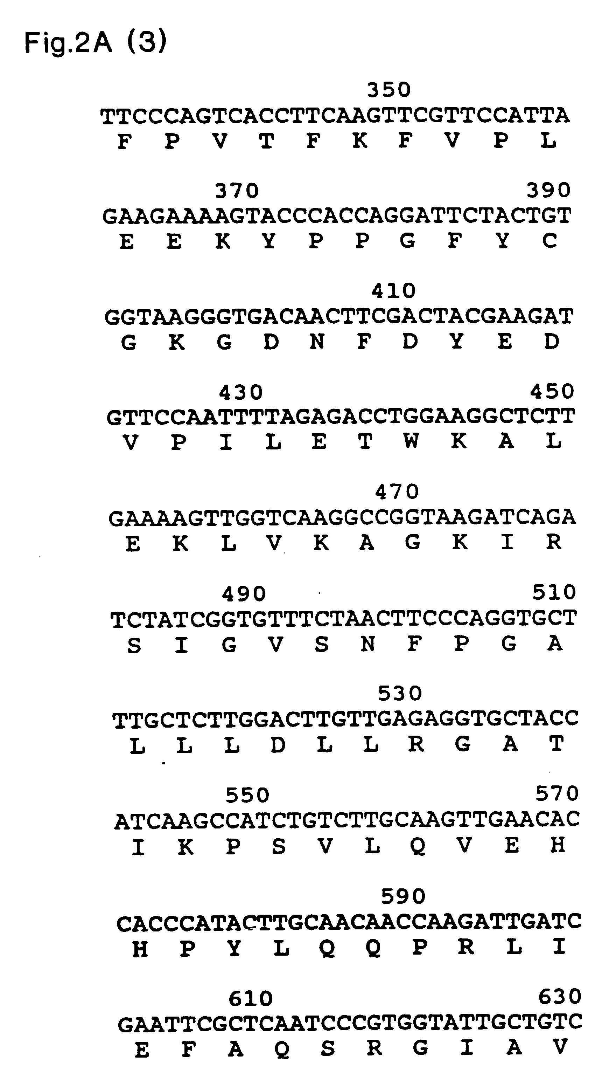

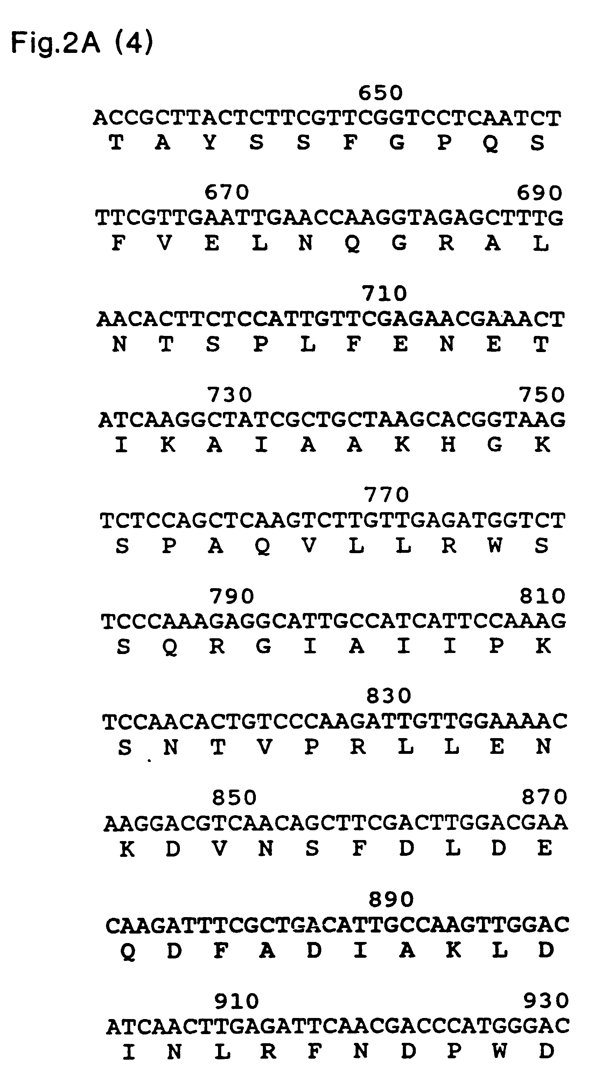

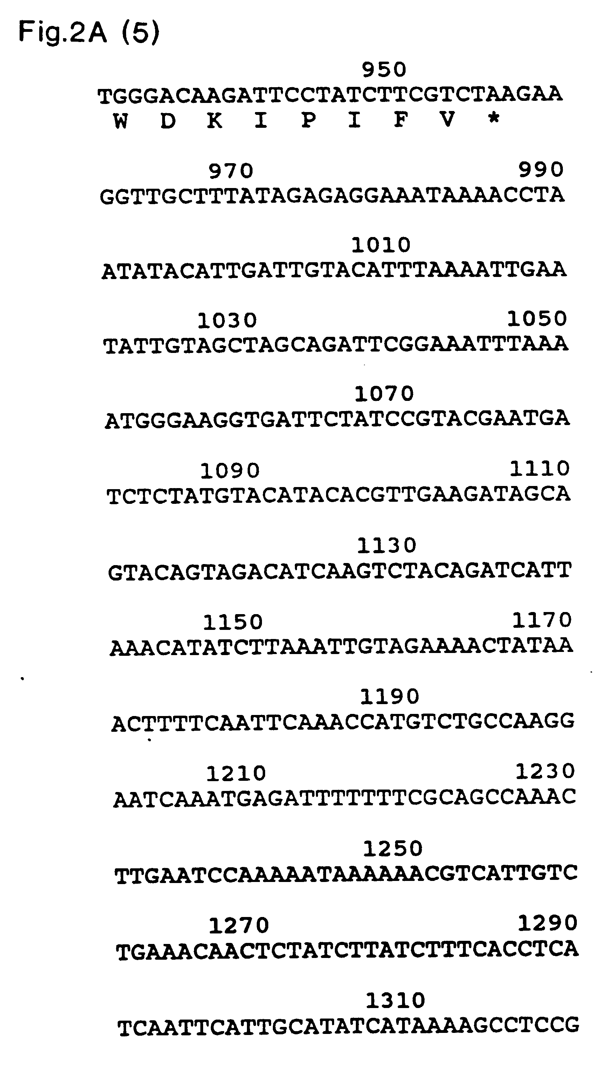

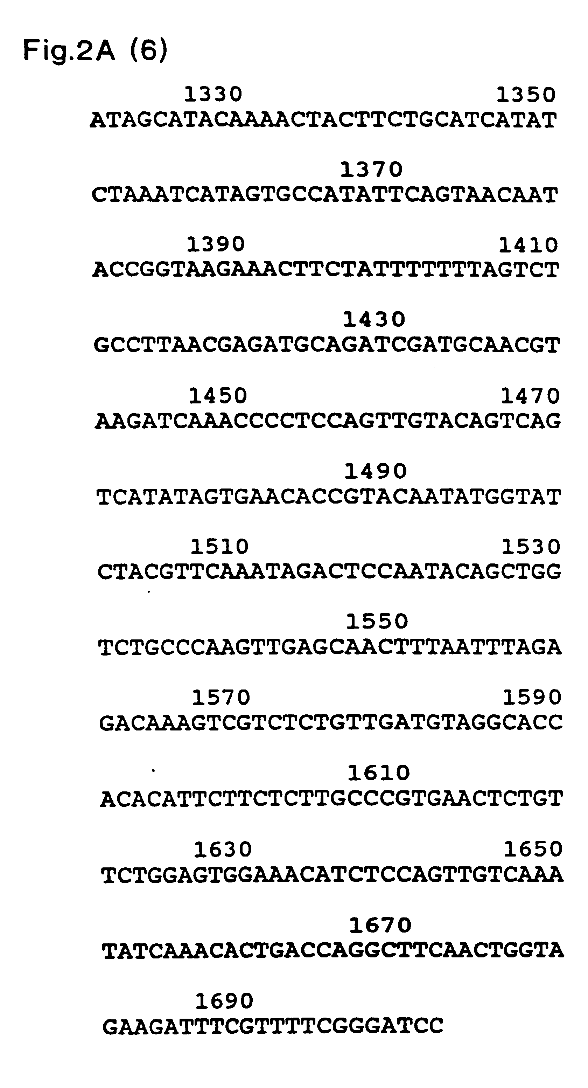

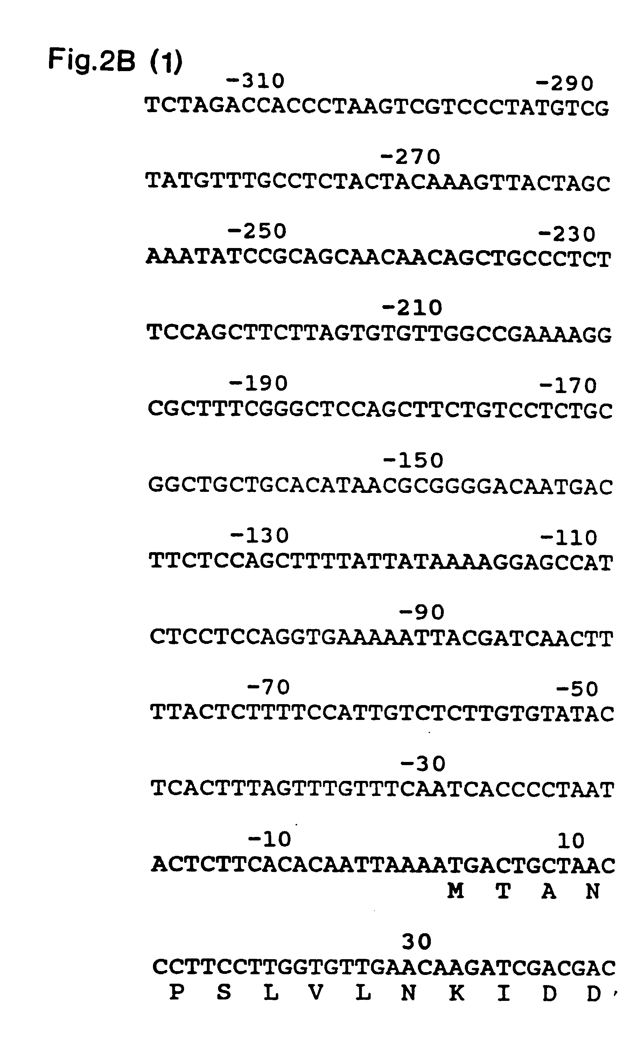

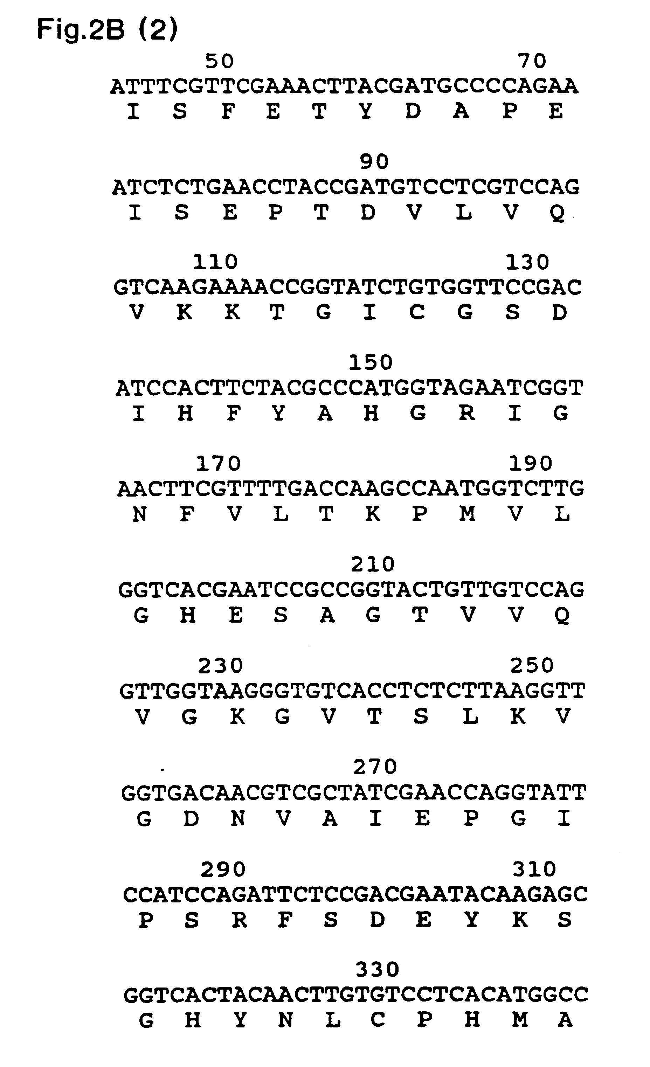

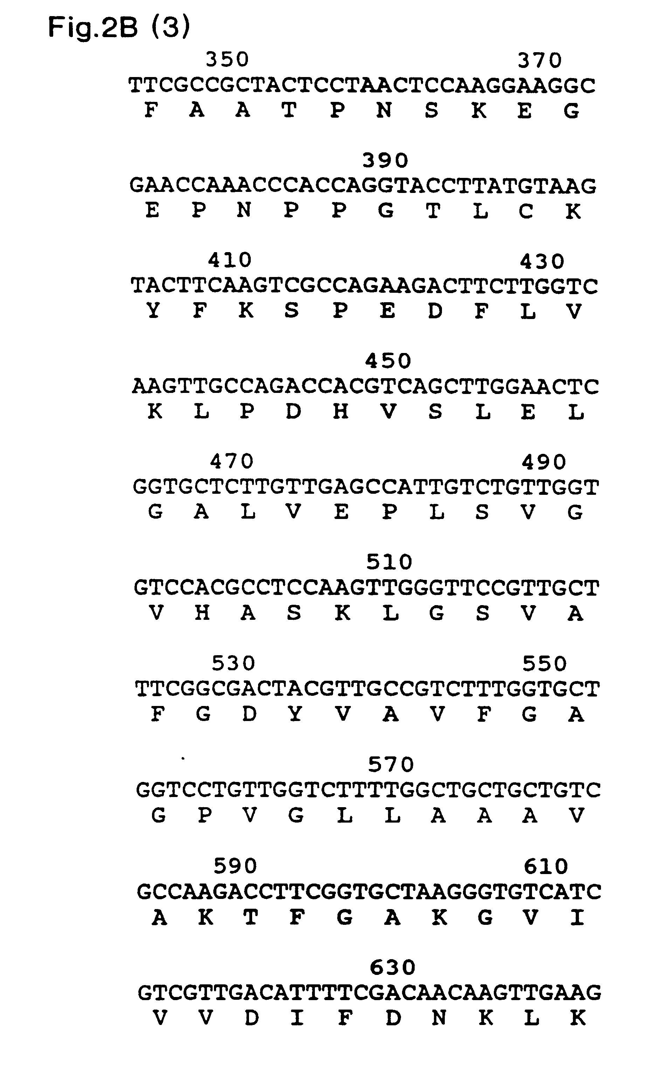

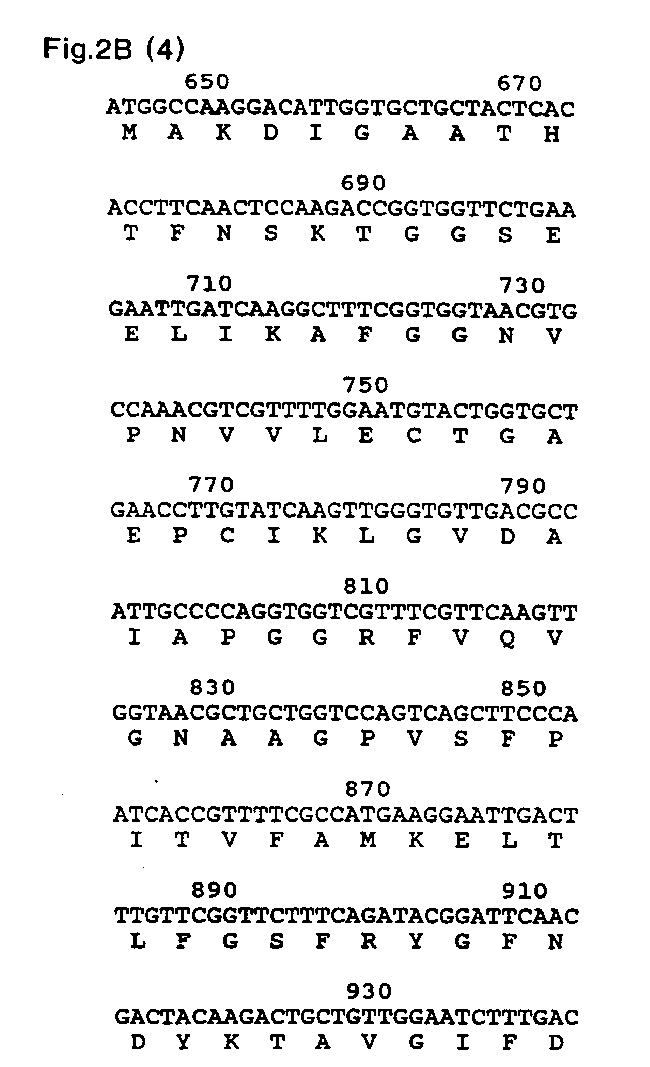

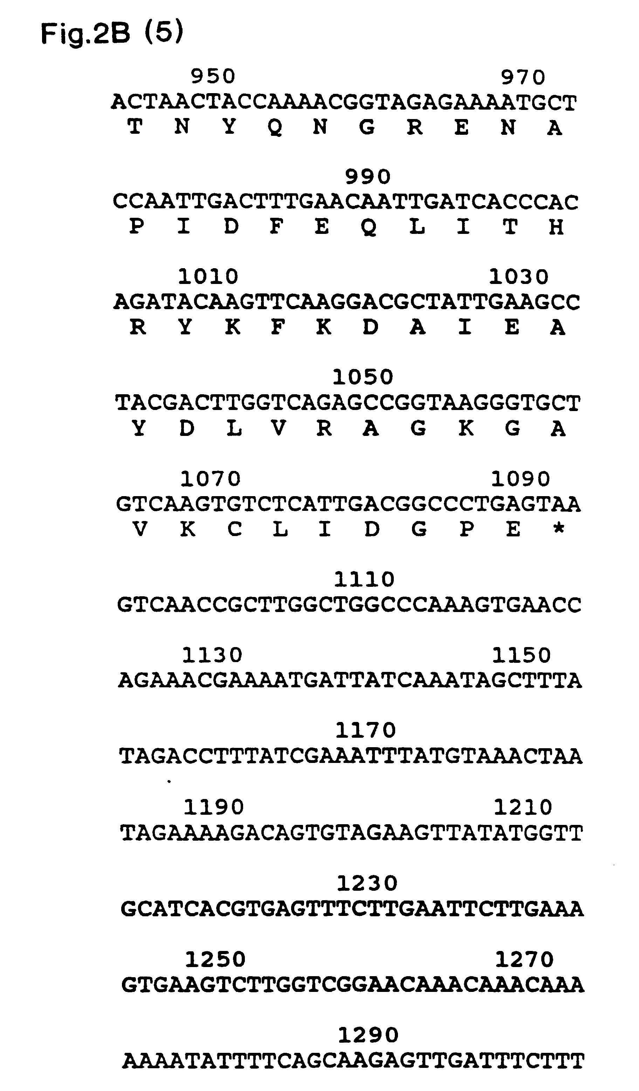

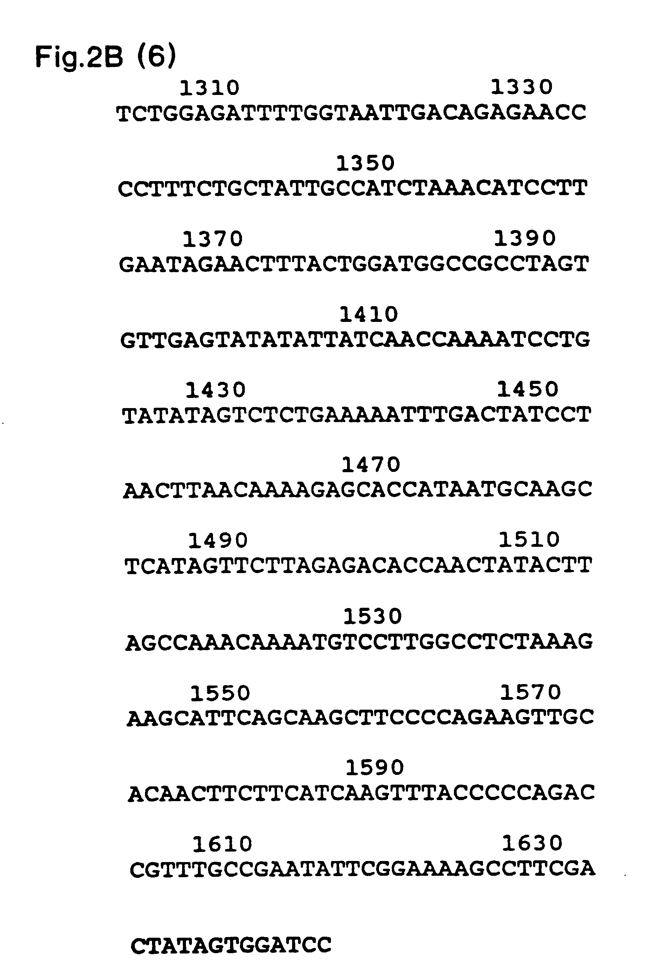

The present inventors succeeded to isolate DNA molecules containing a sequence comprising the structural gene encoding a xylose reductase and a xylitol dehydrogenase respectively. By way of the DNA sequence, which was determined according to standard procedures, the amino acid sequence of both these proteins could be determined for the first time. The complete amino acid sequences as well as the nucleotides sequences of both these proteins are shown in Figures 2A and 2B. As is known to everybody skilled in the art the proteins having the amino acid sequences as shown in Figures 2A and 2B can be encoded not only by the DNA sequences as found in Pichia stipitis CBS5773, but also by using alternative codons provided by the degeneracy of the genetic code. The invention thus is not limited to the DNA sequence as shown in Figure 2, but also comprises any modification yielding the same amino acid sequences.

The DNA sequences according to the present invention may not only be obtained by applying the methods shown below, i.e., by isolating cDNA clones, which further on are used to screen a genomic library, but also may be obtained by other methods of recombinant DNA technology from either natural DNA or cDNA or chemically synthesized DNA or by a combination of two or more of these DNAs. For example, it may be attempted to combine a chemically synthesized 5' region with a cDNA coding for the 3' region or any other combination of the three DNA sources mentioned above.

According to the present invention there are also provided combinations of DNA sequences, which comprise a DNA sequence as discussed above, i.e., a sequence comprising a structural gene coding for a xylose reductase or xylose reductase and xylitol dehydrogenase, and in addition one or more DNA sequences capable of regulating the expression of the structural genes mentioned above in a presumptive host microorganism. DNA sequences capable of regulating the expression of structural genes are well known to those skilled in the art. For example, the DNA sequences discussed above may be combined with promoters, which are connected with the structural genes in order to provide efficient expression. Further DNA sequences capable of regulating the expression may comprise enhancers, termination sequences and polyadenylation signals. Examples for the best known kind of regulating sequences, are shown by the following examples.

In order to express the DNA sequences and/or the combination of DNA sequences according to the present invention efficiently, small modifications of the DNA sequences may be performed, as long as their capability to express a functional enzyme having the desired xylose reductase or xylitol dehydrogenase activity is retained. These modifications may include either variations of the genetic code as discussed above or furthermore small substitutions of the amino acid sequence, as well as deletions and/or insertions, which do not have any detrimental impact on the respective enzyme activity.

In a preferred embodiment the DNA sequence, capable of regulating the expression of the structural gene, is derived from an endogenous gene of the microorganism, in which expression of the DNA sequence is intended. Since, as will be shown below in more detail, Saccharomyces cerevisiae is one of the preferred microorganism to be used in the present invention, there are a multitude of possible regulating sequences known. Some of these well-known sequences have been used to construct expression vectors, as will be shown below in the examples. In the most preferred embodiment the combination of DNA sequences comprises inducible promoters. In this case the expression of xylose reductase and xylitol dehydrogenase can be prevented, as long as desired; expression may be started upon addition of a suitable inducer.

In the most preferred embodiments of the present invention the following Saccharomyces cerevisiae promoters are used to regulate the expression of the genes encoding xylose reductase or xylose reductase and xylitol dehydrogenase: ADH1, ADH2, PDC, GAL1/10.

Depending on the choice of the respective promoter it may be possible to obtain expression levels exceeding that of natural expression of both proteins in their original host organism.

The DNA sequences as well as the combinations of the DNA sequences according to the present invention may be introduced in vector molecules. These molecules may be plasmids, which are suitable for replication in the desired host microorganism and thus should contain a functional origin of replication. Alternatively, it is also possible, to use linear DNA fragments carrying the DNA sequence or combination of DNA sequences according to the present invention or to use circular DNA molecules being devoid of a functional origin of replication. In this case the vector, which is not capable of replication, will be inserted by either homologous or nonhomologous recombination into the host chromosome.

Subject of the present invention are further microorganisms, which have received DNA sequences comprising the inventive DNA sequences or combinations of DNA sequences coding for xylose reductase and xylitol dehydrogenase by recombinant DNA technology.

Preferred microorganisms are selected from a group consisting of yeast of the genera Saccharomyces, Schizosaccharomyces, Schwanniomyces, Kluyveromyces, Pichia, Hansenula, Candida, Debaryomyces, Metschnikowia, Pachysolen or Paecilomyces or bacteria of the genus Zymomonas.

From these organisms the most preferred microorganisms are Saccharomyces cerevisiae and Schizosaccharomyces pombe and Zymomonas.

One of the possible applications of the genetically altered yeast strains described above is the production of biomass. Since the yeast strains having acquired the ability of expressing xylose reductase or xylose reductase and xylitol dehydrogenase are maintaining good fermentation abilities, biomass can be produced most efficiently by use of these inventive yeast strains. The methods for producing biomass are the usual ones, which are known to everybody skilled in the art. The genetically manipulated yeast strains provided in compliance with this invention are also suitable for the production of ethanol. The preferred organisms for use in the production of ethanol by fermentation are the yeasts Saccharomyces cerevisiae and/or Schizosaccharomyces pombe and/or the bacterium Zymomonas.

The preferred carbohydrate in the ethanol production is xylose. Thus, strains of Saccharomyces cerevisiae and/or Schizosaccharomyces pombe and/or Zymomonas being able to ferment xylose are highly advantageous in the production of ethanol. The production of potable spirit or industrial ethanol by use of a genetically manipulated yeast strain according to the present invention can be carried out in a manner known per se. The inventive yeast strains have the ability to ferment concentrated carbohydrate solutions, exhibit high ethanol tolerance and have the ability of producing elevated concentrations of ethanol; they have a high cell viability for repeated recycling and exhibit remarkable pH-and temperature tolerance. In the process of xylose production xylose is formed as a waste product from hydrolysis of xylan, which is the major compound of hemicellulose. Hence it is of great advantage to use xylose for the production of ethanol and/or biomass. The invention is further suitable for the production and isolation of the NAD(P)H linked xylose reductase. Due to the reduction reaction this enzyme is suitable for the delivering or recycling (from NADPH to an NADP⁺) of the corresponding coenzyme especially in bioreactors, for example for the production of amino acids.

A further subject of the present invention is a method for producing the xylose reductase or xylose reductase and xylitol dehydrogenase by cultivating a microorganism according to the present invention under suitable conditions and recovering said enzyme or both of them in a manner known per se. The method thus includes the expression of a DNA sequence or a combination of DNA sequences according to the present invention in a suitable microorganism, cultivating said microorganism under appropriate conditions and isolating the enzyme.

It could be shown, that the level of expression of desired proteins in the inventive microorganisms is enhanced, if the microorganism has been selected for efficient fermentation of xylulose. Thus, is is preferred, to perform the method for reproducing one or both of the proteins using microorganisms, which have been selected accordingly.

Since the present invention provides the cloned genes and the corresponding sequences, the gene products can be overproduced in other organisms, e.g. in yeasts of the genera Saccharomyces, Schizosaccharomyces, Schwanniomyces, Kluyveromyces, Pichia, Hansenula, Candida, Debaryomyces, Metschnikowia, Pachysolen, Paecilomyces or bacteria of the genus Zymomonas. The techniques employed for obtaining expression of the XYL1 (xylose reductase) or XYL1 and XYL2 (xylitol dehydrogenase) gene and the isolation of the active gene product are the usual ones such as promoter-fusion, transformation, integration and selection, and methods of protein isolation, known by the man skilled in the art.

Generally, said microorganisms have received the DNA sequence or combination of DNA sequences via transformation procedures. For each of the possible microorganisms, i.e. the different yeast genera and bacteria of the genus Zymomonas, there are transformation procedures known. The transformation is preferably carried out using a vector, which may be either a linear or circular DNA molecule; in addition, the method can be performed using autonomously replicating or integrative molecules as well. In the case, that the molecule is supposed to integrate into the genome of the respective host, it is preferred, to use a vector containing DNA, which is homologous to the DNA of said intended host microorganism. This measure facilitates homologous recombination.

Further subjects of the present invention are the enzymes produced according to the above described method.

The microorganisms according to the present invention may be used in ethanol manufacturing processes. Since xylose is a readily available source, which normally is considered to be waste, the ethanol manufacturing process according to the present invention provides a possibility for ethanol production of high economical and ecological interest.

The ethanol manufacturing process may be adapted for the production of alcoholic beverages or single cell protein from substrates containing free xylose, which is preferably released by xylanase and/or xylosidase activity from xylan.

According to the present invention there is further provided a method for the production of a desired protein in Pichia stipitis. According to this method a structural gene coding for a desired protein is expressed under control of the 5' regulating region of the XYL1 and/or XYL2 gene from Pichia stipitis and/or the ADH1 promoter of S. cerevisiae and/or the glucoamylase promoter from Schwanniomyces occidentalis. Out of the promoters mentioned before use of the 5' regulating regions of the XYL1 or XYL2 genes is preferred, because these promoters may be induced by adding xylose. Pichia stipitis, when used as a host organism, exhibits the great advantage of having an efficient secretion system. This facilitates an efficient expression not only of proteins, which stay inside the cell, but also of proteins, which are continuously secreted into the medium. A further advantage of the Pichia stipitis expression system is the possibility of using xylose as a substrate. Xylose is a rather inexpensive, readily available nutrient.

The invention will be discussed in detail by way of the following figures and examples.

### BRIEF DESCRIPTION OF THE FIGURES:

Fig. 1
   - A:: restriction map of the DNA fragment encoding the xylose reductase gene (XYL1)
   - E:: EcoR1, H: HindIII, B: BamHI, N: NcoI,
   - P:: PvuII, Ps: Pst1
   - B:: restriction map of the DNA fragment encoding the xylitol dehydrogenase gene (XYL2)
   - Ba:: BamHI, B: BglII, E: EcoRl, X: XbaI, S: SalI
Fig. 2
   A) Nucleotide sequence of the XYL1 structural gene including its 5'- and 3'-flanking sequences and the corresponding amino acid sequence.
   B) Nucleotide sequence of the XYL2 structural gene including its 5'- and 3'-flanking sequences and the corresponding amino acid sequence.
Fig. 3 S. cerevisiae and S. pombe expression vector. Plasmid pRD1 contains both the xylose reductase gene and xylitol dehydrogenase gene under control of their original promoters.
Fig. 4 Fermentation curve of PK4 grown in YNB, 2% xylose medium. The culture was inoculated with 10⁸ cells/ml from a xylose grown preculture. The figure shows xylose consumption and conversion into ethanol with a theoretical maximum yield.
Fig. 5 (1,2) Construction scheme for constructing the vector pBRPGAM. For constructing this vector, the 3.8 kb EcoRI-PvuII-fragment from pBRSwARSGAM containing the functional GAM promoter and base pairs 1 to 208 of the coding GAM sequence was ligated to the small EcoRI-PvuII-fragment of pBR322.
Fig. 6 (1,2) Construction scheme for constructing the vector pBRGC1. For constructing this vector, the 3.4 kb PvuII-fragment of pCT603 containing the structural gene for xylose starting with nucleotide + 122 was inserted into the PvuII site of vector pBRPGAM.
Fig. 7 (1,2) Construction scheme for constructing the vector pMPGC1-2. The 6.5 kb BamHI-PstI-fragment of pBRGC1 containing the cellulase gene under control of the GAM promoter was ligated with the large BamHI-PstI-fragment of pCJD5-1.

### EXAMPLES

### Materials and Methods

### I. Microorganisms and cultivation

Yeast strains:
1. S. cerevisiae:
   a) XJB3-1B (MAT α, met6, gal2) was obtained from the Yeast Genetic Stock Center (see Catalogue of the Yeast Genetic Stock Center, 6. edition, 1987).
   b) GRF18 (MAT α, leu2-3, leu2-112, his3-11, his3-15) was obtained from G.R. Fink (DSM 3796).
   c) AH22 (MATa, can1, his4-519, leu2-3, leu2-112) was obtained from A. Hinnen (DSM 3820).
2. Schizosaccharomyces pombe (leu1-32, his5-303) (DSM 3796).
3. P. stipitis CBS5773 (DSM 5855) was obtained from Centraalbureau voor Schimmelcultures, Yeast Division, Delft, The Netherlands.

Yeast strains were grown at 30°C in YP medium (1% yeast extract, 2% bacto pepton) or in 0.67% Difco yeast nitrogen base (YNB) without amino acids, optionally supplemented with appropriate amino acids. Media were supplied with either 2% xylose or 2% glucose. The yeasts were transformed according to Dohmen et al. (1989).

E. coli strains:
1. DH5αF' (supplied by BRL company, Eggenstein, FRG)
2. HB101 (DSM 3788) (Bolivar et al., 1977).

E. coli strains were grown at 37°C in rich medium (LB-medium, Maniatis et al., 1982). The medium was supplemented with penicillin G (100 µg/ml) when selecting for transformants. E. coli transformation was carried out as described by Maniatis (1982).

### II. Purification of the XR and XDH proteins from P. stipitis

Cells were grown under induced conditions to exponential growth phase. To prepare cell-free extracts cells were harvested by centrifugation and were broken with glass beads in a Braun homogenizer using 0.1 M Tris-HCl buffer (pH 7.0). The supernatant obtained following 1 h centrifugation of the crude extract (150000 x g) was loaded on an affinity chromatography column (Affi-Gel Blue, 60x50 mm) preequilibrated with 5 mM NaPO₄ buffer (pH 6.8) and eluted with 1.5 mM NAD. The fractions containing XR and XDH activity were pooled and dialysed against 20 mM Tris-HCl (pH 7.5). The dialysate was subsequently applied to a DEAE-Sephacel anion exchange column preequilibrated with 20 mM Tris-HCl (pH 7.5). Proteins were eluted with a linear gradient (20-250 mM Tris-HCl, pH 7.5). Fractions containing the highest activity were pooled, concentrated and loaded on a SDS-PAA-gel. After running the gel was stained with 0.1 M KCl and the XR- and XDH-proteinbands were cut out, both proteins were separately eluted from the polyacrylamide gel by dialysis using 20 mM NaPO₄ (pH 8.0), 0.1% SDS; subsequently the dialysate was concentrated. All buffers contained 0.2 mM DTT (Dithiothreitol) and 0.4 mM PMSF (Phenylmethansulfonylfluoride).

### III. Preparation of antisera

Mice were given intraperitoneal injections of 2-5 µg protein in Freund complete adjuvant. Two weeks later the same amount of protein in Freund incomplete adjuvant was injected; a third injection was administered another 2 weeks later omitting Freund adjuvant. Antiserum was harvested six weeks after the first injection.

### IV. Immunoscreening

Antisera raised in mice against purified P. stipitis xylose reductase (XR) and xylitol dehydrogenase (XDH) protein, respectively, were used for screening the cDNA library following the procedure of Huynh et al. (1985). The antisera were diluted 10.000-fold. Bound antibodies were visualized using an alkaline phosphatase-conjugated goat anti-mouse immunoglobulin antibody, followed by a colour development reaction with the phosphatase substrate 5-bromo-4-chloro-3-indolyl phosphate (BCIP) in combination with nitro blue tetrazolium (NBT).

### V. Isolation of RNA

All procedures were carried out at 0 to 4°C, if not indicated otherwise. All solutions and materials were sterilized if possible. P. stipitis cells were grown to midexponential phase in the presence of xylose. Yeast cells were harvested by centrifugation, washed twice with buffer 1 (20 mM NaCl, 10 mM MgCl₂, 100 mM Tris-HCl, ph 7.6) and suspended in the same buffer (1.25 ml/g cells). 1/10 volume phenol, 200 µg/ml heparin, 100 µg/ml cycloheximid and 0.4% SDS were added. Disruption of the cells was carried out by shaking with glass beads (0.45 - 0.5 mm) in a ratio of glass beads to suspension of 1:1 (v/v) in a Braun homogenizer (Braun, Melsungen). Two volumes of buffer 2 (buffer 1 containing 100 µg/ml heparin, 50 µg/ml cycloheximid, 2% SDS) were added to the homogenate, cell debris were removed by centrifugation (10000 x g, 10 min). The solution was extracted three to five times with phenol/chloroform (1:1), once with chloroform/ isoamylalcohol (24:1). The nucleic acid was precipitated by incubating the aqueous phase with 2,5 volume of ethanol in the presence of 0.2 M NaCl over night at -20°C. The precipitate was solubilized in buffer 3 (0.1 M NaCl, 1 mM EDTA, 10 mM Tris-HCl, pH 7.5); SDS and LiCl were added to a final concentration of 0.1% and 4 M, respectively. The RNA was precipitated over night at +4°C. The pellet was washed twice with 70% ethanol and suspended in sterilized H₂O before use. RNA was stored at -70°C as an ethanol precipitate.

### VI. Enzyme assays

Activities of xylose reductase (EC. 1.1.1.21) and xylitol dehydrogenase (EC. 1.1.1.9) were measured as described by Bruinenberg et al. (1983). Protein was determined with the micro biuret method according to Zamenhoff (1957) using bovine serum albumin as standard.

### VII. Gelelectrophoresis

SDS gelelectrophoresis was carried out in 10% PAA according to Laemmli (1970).

### VIII. Immunoblotting

Detection of antigenic proteins was carried out as described by Towbin et al. (1979) using the antisera obtained from mice. The proteins were transferred to a polyvinylidene difluoride microporous membran (Millipore, Immobilon PVDF) and were visualized by a phosphatase-coupled colour reaction (Blake et al., 1984). Alkaline phosphatase conjugated to goat anti-mouse IgG was obtained from Jackson Immunoresearch Lab. (Avondale, USA).

### IX. DNA-sequence analysis

XYL1 and XYL2 genomic DNA as well as the respective cDNAs were subcloned in pT7T3-18U (Pharmacia). Fragments obtained by partial digestion using Exonuclease III (Henikoff, 1984) were analysed and sequencing was carried out by the dideoxy method of Sanger et al. (1977) using the T7-Sequencing™kit (Pharmacia). Both strands were completely determined by obtaining overlapping sequences at every junction.

### X. Construction of a P. stipitis CBS 5773 (DSM 5855) cDNA library

Total RNA was extracted according to the method described above. Poly (A)⁺-RNA was prepared by chromatography on an oligo(dT)-cellulose column using essentially the method described by Maniatis et al. (1982). A cDNA library in λgt11 was prepared by the method of Gubler and Hoffman (1983) using a cDNA synthesis kit (Pharmacia) and in vitro packaging of the recombinant λgt11-DNA according to Hohn and Murray (1974) using the in vitro packaging kit supplied by Boehringer, Mannheim (FRG).

### XI. Preparation of crude extracts

Cells were grown to late exponential growth phase and washed twice in buffer (10 mM potassium phosphate, pH 7.0, 1 mM EDTA, 5 mM β-mercapto ethanol). Cells were broken in an Braun homogenizer with an equal volume of glass beads. The supernatant resulting from 5 min centrifugation at 10000 g was used in enzyme assays. Extracts for Western blot analysis were boiled in 1% SDS, 5% β-mercapto ethanol, 10 mM potassium phosphate pH 7.0 and 10% glycerol.

### EXAMPLE 1:

### Isolation of the xylose reductase (XYL1) and xylitol dehydrogenase (XYL2) genes.

A λgt11 cDNA library constructed from poly (A)⁺-RNA of P. stipitis was screened with mouse polyclonal antibodies raised against the purified xylose reductase (XR) and xylitol dehydrogenase (XDH) proteins, respectively. Among 110.000 recombinant clones of the amplified cDNA library containing about 55.000 primary clones, seven identical XYL1 clones and three identical XYL2 clones were identified and purified. The analysis of the insert size revealed that the XYL1 clones contain two EcoR1 fragments (0.6 kb and 0.4 kb), whereas the XYL2 clones contain a single 0.55 kb EcoR1 fragment. The respective EcoR1 fragments of the λgt11 clones were subcloned into the single EcoR1 site of plasmid pT7T3-18U (Pharmacia) resulting in plasmids pXRa (containing the 0.4 kb EcoR1 fragment of the XYL1 clone), pXRb (containing the 0.6 kb EcoR1 fragment of the XYL1 clone) and pXDH (containing the 0.55 kb EcoR1 fragment of the XYL2 clone).

These plasmids were used as a radioactive probe to screen a P. stipitis genomic library, which was constructed by ligation of partial Sau3A digested P. stipitis DNA into the single BamH1 site of the S. cerevisiae - E. coli shuttle vector YEp13 (Broach et al., 1979) resulting in about 60.000 independent clones after transformation of E. coli HB101.

Two plasmids, namely pR1 and pD1 could be isolated and were used for transformation of S. cerevisiae GRF18. XR activity could be detected in the crude extracts of the transformants containing pR1, whereas transformants carrying pD1 yielded crude extracts exhibiting XDH activity. In a mitotic stability test (Beggs 1978) the LEU2 marker and the XR or XDH gene cosegregated, indicating that pR1 and pD1 harbour the functional XYL1 (xylose reductase) and XYL2 (xylitol dehydrogenase) gene, respectively.

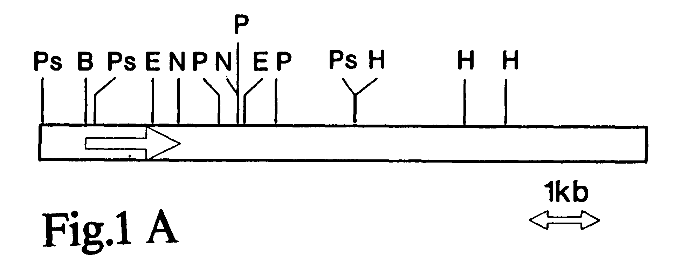

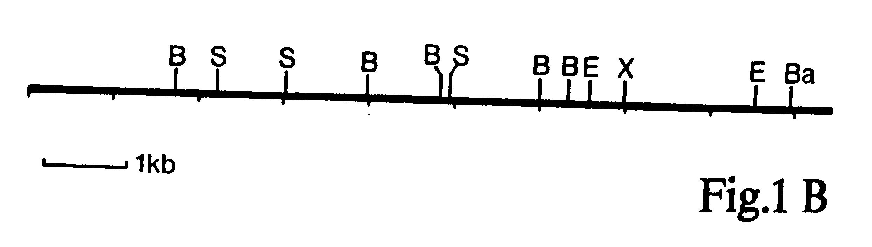

The plasmids pR1 and pD1 were subjected to restriction enzyme analysis yielding the map of restriction sites of the XYL1 (Fig. 1A) and XYL2 (Fig. 1B) genes, respectively.

Further subcloning experiments revealed that the XYL1 gene is encoded within a 2.04 kb BamHI genomic fragment. One of the BamHI sites is not present in the original plasmid pR1. It must have been generated during subcloning. The XYL2 gene is encoded within a 1.95 kb BamHI-XbaI fragment. The 2.04 BamHI fragment and the 1.95 kb BamHI-XbaI fragment were subcloned into the multiple cloning site of pT7T3-18U resulting in pR2 and pD2, respectively, and subjected to DNA sequence analysis. The DNA sequence of the structural gene and of the 5' and 3' non-coding region of the XYL1 and the XYL2 gene is depicted in Fig. 2A and Fig. 2B, respectively.

The DNA sequence of the XYL1 gene contains an open reading frames of 954 bp (318 amino acids) whereas that of the XYL2 gene comprises an ORF of 1089 bp (363 amino acids).

The amino acids deduced from the open reading frames are shown in Fig. 2A and Fig. 2B. The sequences correspond to an XR polypeptide and an XDH polypeptide with a calculated molecular weight of 35922 and 38526 D, respectively.

### EXAMPLE 2

### Expression of both the xylose reductase and xylitol dehydrogenase gene in S. cerevisiae.

Saccharomyces cerevisiae was contransformed with pR1 and pD1. The highest measurable activities of XR and XDH in S. cerevisiae transformed accordingly correspond to 50% of the activities of both enzymes measurable in P. stipitis crude extracts. In S. cerevisiae the genes were expressed in YNB medium containing 2% glucose as a sole carbon source, whereas in P. stipitis expression of both genes is repressed by glucose and induced by xylose. Taking into account the copy number of 10 of YEp13 in S. cerevisiae and assuming a gene dosage dependent expression one can conclude that the Pichia promoters are 20 times less efficient in S. cerevisiae than in P. stipitis.

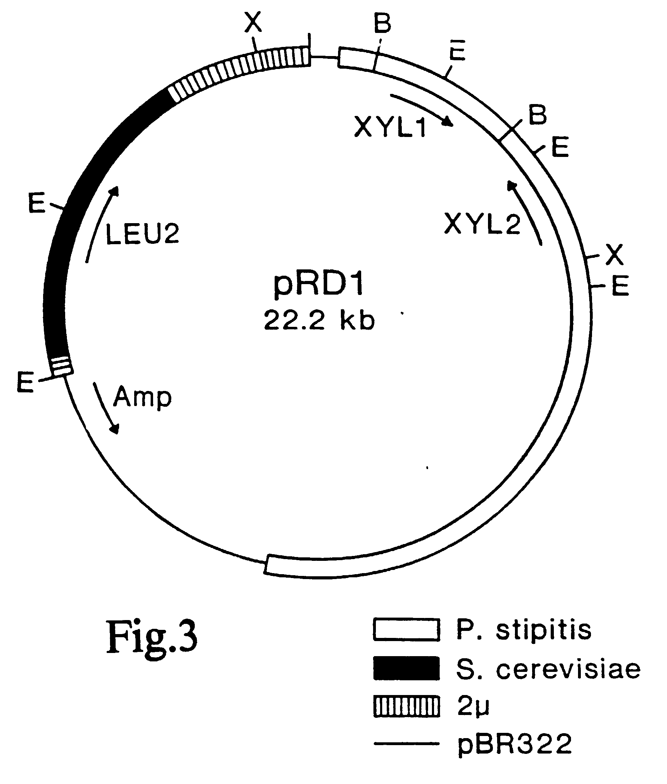

Furthermore, a plasmid harbouring both the XYL1 and XYL2 gene including their original Pichia promoters was constructed (Fig. 3). This plasmid pRD1 was used to transform strain GRF18 by selection on leucine resulting in the transformant PK1. However, expression was not improved compared to cotransformation with separate plasmids.

### EXAMPLE 3

### Construction of an integrative vector containing the XYL2 gene under control of different promoters

Different expression vectors using different promoters for integration and gene expression in S. cerevisiae were constructed. For example the XYL2 gene was fused to the ADH1 promoter followed by homologous integration into the HIS3 locus of S. cerevisiae. The strategy employed was as follows: The 1.5 kb XbaI/ EcoR1 fragment containing the xylitol dehydrogenase gene XYL2 was inserted into the multiple cloning site of pT7T3-18U (Pharmacia) resulting in plasmid pXDH. To eliminate the promoter region of the XYL2 gene this plasmid was linearized with XbaI (restriction site 318 bp upstream of the initiator ATG codon) and with PstI to protect the 3' end of the plasmid DNA. The linear plasmid was treated with exonuclease III and subsequently with S1 nuclease to remove the DNA between the XbaI site and the XYL2 structural gene. The deleted DNA molecules were recircularized, cloned in E. coli and the extent of deletion was determined by dideoxy sequencing. In one of the modified pXDH plasmids the 5' untranslated region and the four N-terminal amino acids were deleted. However, a new inframe ATG initiation codon was created due to the SphI site from the multiple cloning site. A BamHI linker was inserted into the HindIII site of the multiple cloning site. Subsequently, a 1.5 kb BamHI fragment carrying the XYL2 gene could be subcloned into vector pT7T3-18U resulting in additional restriction sites in front of the ATG initiation codon. The newly created 5' region is as follows: ATG CCT TGG TGT...(deletion of original amino acid 2,3 and 4).

To complete the 3' untranslated region of the XYL2 gene a 440 bp EcoRI fragment, was inserted into the single EcoRI site of the 1.5 kb fragment subcloned in pT7T3-18U. This 440 bp fragment was obtained by subcloning the 440 bp EcoR1-BamHI fragments (see Fig. 1B) into another pT7T3-18U, removing the BamHI site by cutting with BamHI and subsequent filling-in with Klenow polymerase. The 3' untranslated region could thus be isolated as 440 bp EcoR1 fragment. In the single BamHI site arranged near the 5' terminus of the XYL2 gene, which is provided by the polylinker region, the 1.8 kb BamHI fragment harbouring the S. cerevisiae His3 gene derived from plasmid YEp6 (Struhl et al. 1979) was inserted. To remove one of the two BamHI sites the resultant plasmid was cut with SalI and XhoI and subsequently recircularized. The resulting plasmid pXDH-HIS3 contains one suitable BamHI site in front of the ATG initiation codon in which the 1.5 kb BamHI fragment, containing the ADH1-promoter (Ammerer, 1983) of S. cerevisiae can be inserted.

Since this plasmid does not contain any autonomous replicating sequence for S. cerevisiae this plasmid can be used for homologous integration (Orr-Weaver et al. 1981) into the HIS3 locus of any S. cerevisiae strain.

In our integration experiments we used a mutagenized XJB3-1B strain called PUA6-1, which was isolated according the protocol of Porep, (1987) and Ciriacy, (1986). The resulting integrant PK2 is expressing the XYL2 gene under control of the ADH1 promoter leading to an active gene product.

### EXAMPLE 4

### Construction of S. cerevisiae and S. pombe integrants expressing both the XYL1 and XYL2 gene.

To eliminate the promoter region of the XYL1 gene plasmid pR2 containing the XYL1 gene on a 2,04 kb BamHI fragment was linearized with XbaI ( restriction site 362 bp upstream of the translation initiation ATG codon) and cleaved with SphI to protect the 3' end of the plasmid DNA. The linear plasmid was treated with exonuclease III and subsequently with S1 nuclease to remove the DNA between the XbaI site and the XYL1 structural gene. The deleted DNA molecules were recircularized, cloned in E. coli and the extent of deletion was determined by dideoxy sequencing. In one of the modified pR2 plasmids the 5' untranslated region was exactly deleted.

The structural gene was subcloned as a HindIII-BamHI fragment into the corresponding sites of YIp366 (Myers et al. 1986). In addition the ADH1 promoter was subcloned into the HindIII site by blunt end ligation resulting in plasmid pXR-LEU2. Since this plasmid does not contain any autonomous replicating sequence for S. cerevisiae this plasmid can be used for homologous integration (Orr-Weaver et al. 1981) into the LEU2 locus of any S. cerevisiae strain, e.g. strain PK2. The resulting integrant PK3 is expressing both the XYL1 and XYL2 genes under control of the ADH1 promoter leading to active gene products. For expression studies in Schizosaccharomyces, S. pombe was transformed with both plasmids pXDH-HIS3 and pXR-LEU2 selecting for histidine and leucine. After extensive screening of the transformants for growth on xylose one transformant called AS1 could be isolated expressing both the XYL1 and XYL2 gene under control of the ADH1 promoters.

In the same manner other S. cerevisiae promoters, e.g. pyruvate decarboxylase (PDC) promoter (Kellermann & Hollenberg, 1988), alcoholdehydrogenase 2 (ADH2) promoter (Russell et al., 1983) or the galactokinase (GAL1/10) promoter from plasmid pBM272, which is derived from plasmid pBM150 (Johnston and Davis, 1984) by introducing a HindIII site immediately following the BamHI site, led to expression of active XYL1 and XYL2 gene product in S. cerevisiae.

In another set of experiments two suitable restriction sites BamHI (position -9) and SalI (position - 15) were introduced just in front of the XYL1 and XYL2 genes.

XYL1:5'attcttttctaGTCGACGGATCCAAGATGCCTTCTATT
...TAA terminator3'

XYL2:5′cccctaatactGTCGACGGATCCAAGATGACTGCTAAC
...TAA terminator3′

These modifiations were introduced by site directed mutagenesis of the 5' region using the site directed mutagenesis kit supplied by Amersham according to the instructions of the manufacturer. These restriction sites offer the possibility to fuse any promoter just in front of the ATG initiation codon. In addition the gene under control of a desired promoter can be isolated as a well defined fragment for insertion into sequences suitable for homologous integration.

For industrial or commercial purposes it is desirable to construct stable production strains of S. cerevisiae and/or S. pombe. Therefore both genes under control of the constitutive ADH1 promoter were integrated without any bacterial sequence into the chromosome of S. cerevisiae strain PUA6-1 via homologous integration (Orr-Weaver et al.1981). Integration into the HO homothallism gene (Russel et al. 1986), ARS-sequence (Stinchcomb et al., 1978) or into the ADH4 gene (Paquin et al., 1986) by cotransformation with pJW6 (Fogel and Welch, 1982) is preferred resulting in strains PK3(HO), PK3(ARS) and PK3(ADH4). In the case of S. pombe the integration mainly occurs via illegitimate recombination. Hence only a few of the S. pombe integrants exhibit XR and XDH activities and have the same fermentation and growth properties as the wild type.

The S. cerevisiae integrants PK3, PK3(HO), PK3(ARS) and PK3(ADH4) may be improved for efficient assimilation of xylulose.

### EXAMPLE 5

### Isolation of a S. cerevisiaemutant efficiently assimilating xylulose.

S. cerevisiae strain XJB3-1B grows slowly on media containing xylulose as a sole carbon source (doubling time 10 hours). According to a protocol described by Porep (Porep, 1987) a mutant, PUA3, was isolated, which utilized xylulose more efficiently than wild type S. cerevisiae strains, resulting in a doubling time of approximately four hours for growth on xylulose as a sole carbon source.

Mutant strain PUA3 also converts xylulose into ethanol in the absence of respiration (Porep, 1987). In order to obtain the PUA genotype in combination with an auxiliary marker (LEU2) useful in yeast transformation, strain PUA3 was crossed to AH22 (leu2 his4). From a sporulating culture of the AH22xPUA3 diploid meiotic spore progenies were isolated which were leu2 and had the ability of efficient xylulose-utilization as observed in the original mutant, PUA3. In an analogous experiment the PUA genotype was combined with leu2 and his3 auxiliary markers by crossing strain GRF18 and PUA strain and subsequent meiotic spore isolation. This resulted in strain PUA6-1 which was PUA leu2 his3.

### EXAMPLE 6

### Isolation of a S. cerevisiaemutant efficiently converting xylose into ethanol.

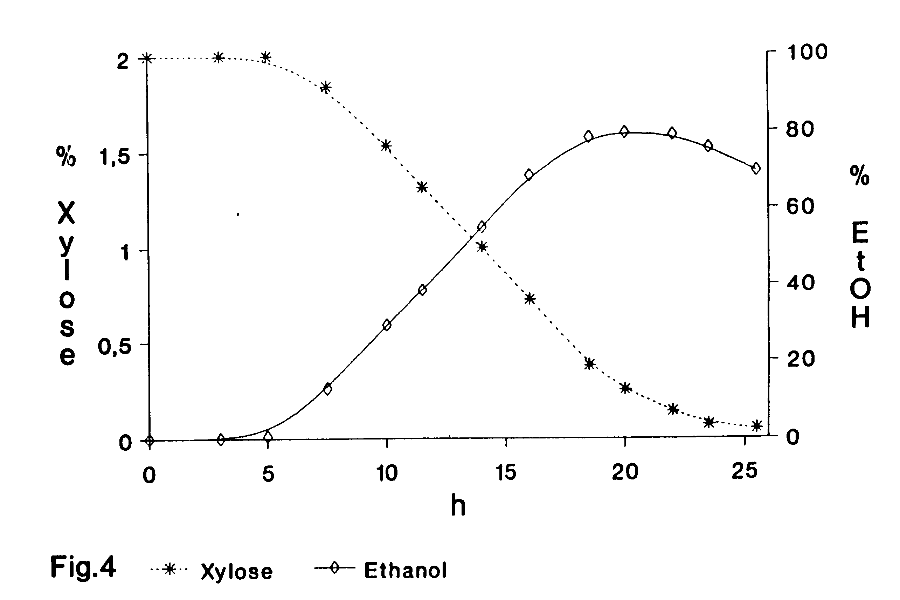

Strain PUA6-1 containing the XYL1 and XYL2 genes chromosomally integrated (See Examples 3 and 4) was able to grow on xylose as a sole carbon source whereas the untransformed PUA6-1 strain was completely negative on YNB xylose media. Doubling time of the transformant strain PK3 was 4 hours on YNB 1% xylose (for comparison, doubling time on YNB 1% glucose: 2 hours). Since ethanol production was inefficient in this strain when grown on xylose and no xylose growth was observed in the absence of respiration a mutant strain with improved capability in converting xylose to ethanol was selected as follows: 10⁸ PK3 cells were mutagenized with UV (254 nm) using conditions allowing 20% to 40% of the cells survival. The surviving cells were grown for approximately 30 generations in YNB 2% xylose liquid media. After plating on xylose solid media isolates were obtained which grow significantly faster than the parent strain PK3. One isolate was further propagated and used for selection of a mutant able to grow on YNB 2% xylose plates supplemented with 2 mg/l antimycin A in order to block respiratory metabolism. This procedure yielded a mutant (PK4) which was able to convert xylose significantly more efficiently to ethanol than the original transformant strain PK3. A typical xylose fermentation protocol is depicted in Fig. 4. The ethanol yield was approximately 40% of the initial xylose. This yield corresponds to approximately 80% of the theoretical maximum yield of ethanol from xylose conversion.

### EXAMPLE 7

### Expression of heterologous genes in Pichia stipitis

Following UV mutagenesis of Pichia stipitis strain CBS 5773 (DSM 5855) a trp5 mutant was isolated. The trp5 mutation was identified by examining indol accumulation according to Hagedorn and Ciriacy (Hagedorn and Ciriacy, 1989).

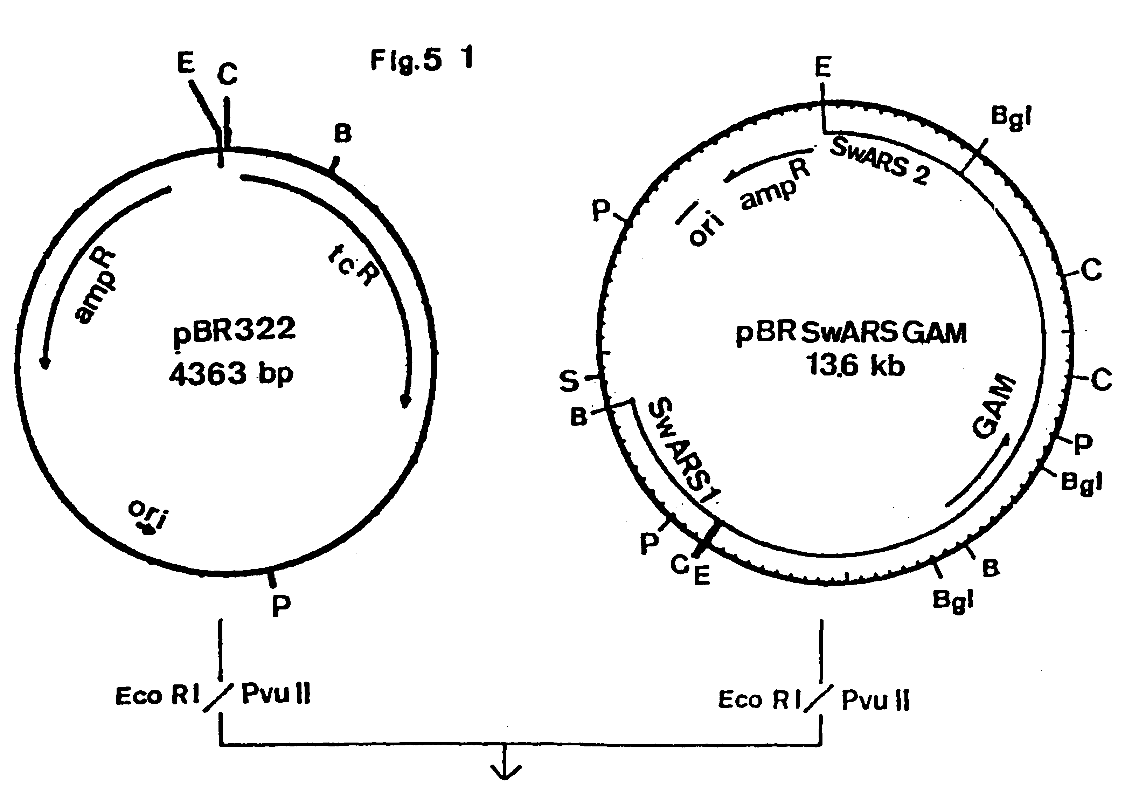

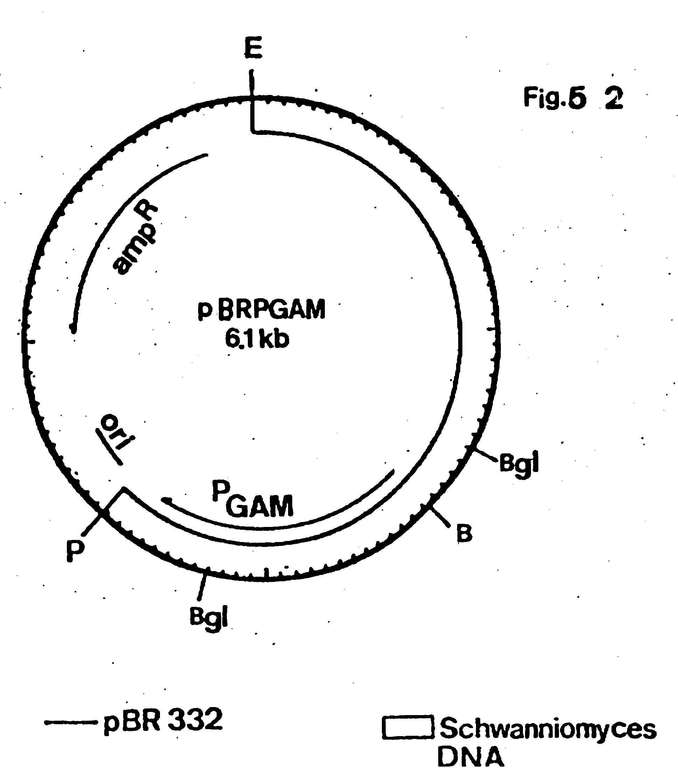

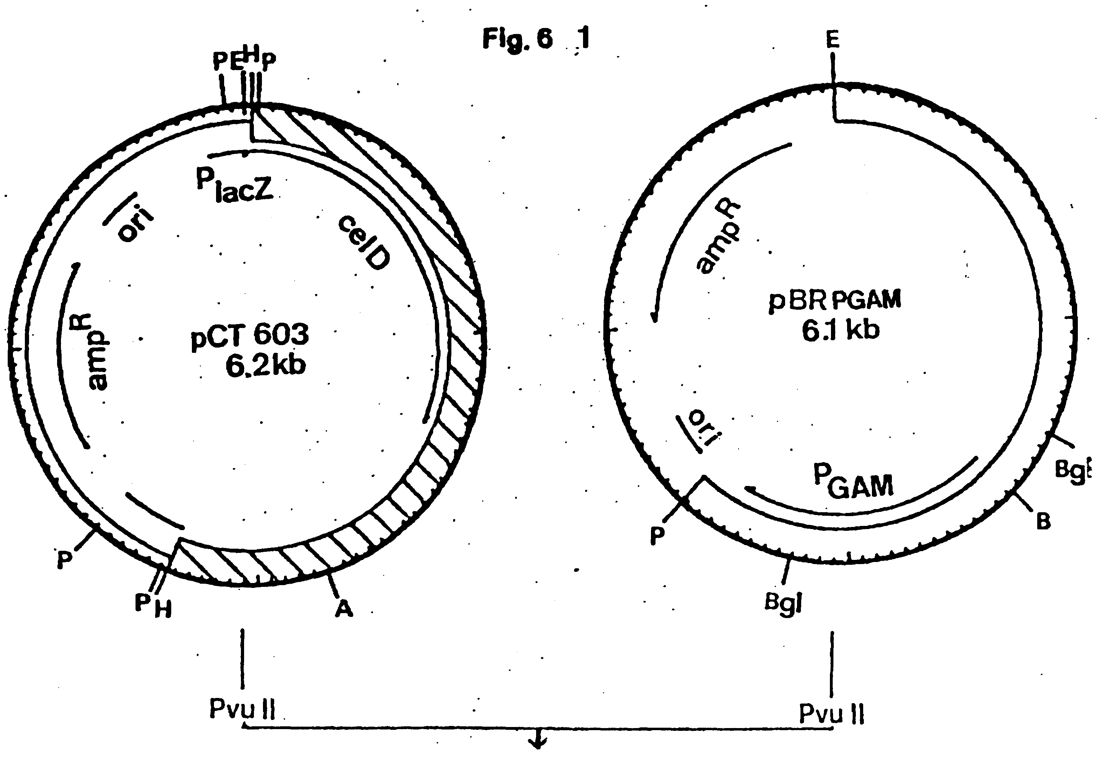

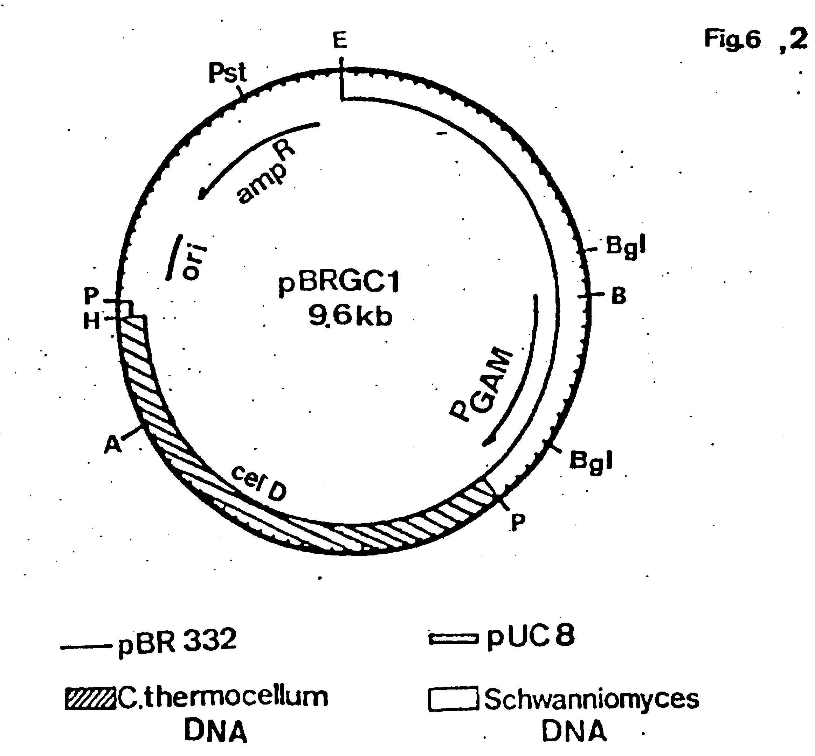

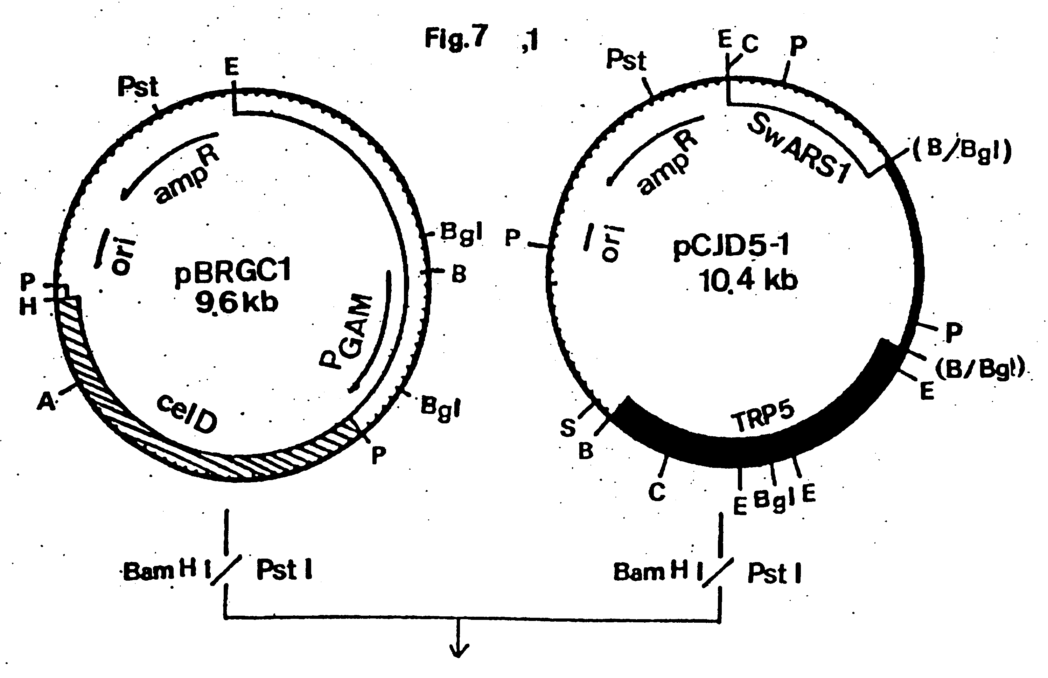

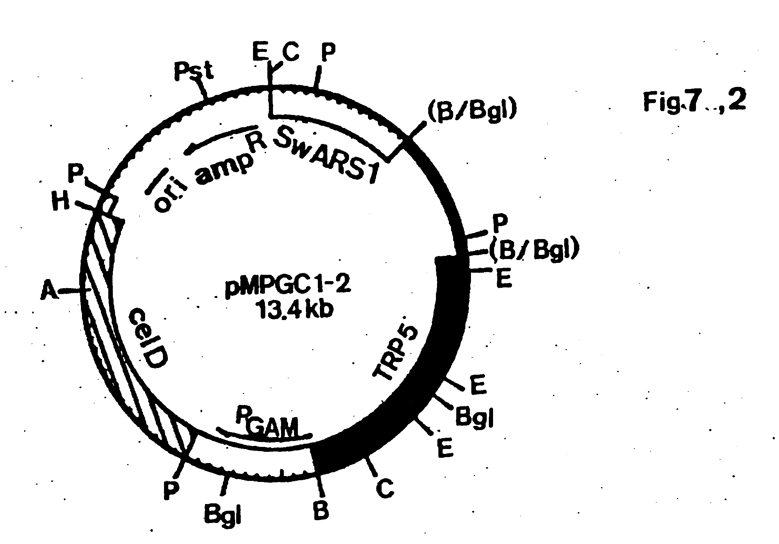

For expression in Pichia stipitis plasmids were constructed which contain a replicon from Schwanniomyces occidentalis (SwARS1), the TRP5-gene from S. cerevisiae (Dohmen et al., 1989) as a selective marker and in addition a glucoamylase(GAM)-cellulase (celD) gene fusion under control of the glucoamylase promoter. In a first step the 3.8 kb EcoRI-PvuII-fragment from plasmid pBRSwARSGAM (Fig. 5, described in EP 89 107 780) was isolated and inserted into the 2296 bp EcoRI-PvuII-fragment from pBR322 carrying the ampicillin resistance gene and the bacterial origin of replication, resulting in plasmid pBRGAM (Fig. 5). In addition to pBR322 derived sequences this plasmid carries 3.6 kb derived from the 5' noncoding region of the glucoamylase gene from Schwanniomyces occidentalis and nucleotides 1 to 208 coding for the N-terminal part including the signal sequence of the glucoamylase. Subsequently, a 3.4 kb PvuII-fragment derived from plasmid pCT603 (Joliff et al., 1986) containing the coding region of the celD-genes from Clostridium thermocellum with the exception of 120 bp (corresponding to 40 amino acids) starting with the 5' terminus of the coding region was inserted into the PvuII site of the pBRGAM resulting in pBRGC1 (Fig. 6). For construction of a P. stipitis expression vector plasmid pCJD5-1 (EP 87 110 370.1) was cleaved with BamHI/PstI and ligated with a 6.5 kb BamHI-PstI-fragment from pBRGC1. The resulting plasmid was termed pMPGC1-2 (Fig. 7). The above described P. stipitis mutant trp5 was transformed with pMPGC1-2 and the transformants were identified by their capability to grow on medium free of tryptophan (tryptophan prototrophy). Transformants were examined for cellulase activity using the congo red assay (Teather & Wood, 1982). The transformants constitutively produce active cellulase (endoglucanase D) of Clostridium thermocellum, which is secreted into the media, indicating, that the promoter and the signal sequence encoded by the glucoamylase gene may control expression of a heterologous gene and secretion of the gene product into the medium.

Subsequently plasmid pMPGC1-2 was modified in order to substitute the glucoamylase promotor either by the S. cerevisiae ADH1-promoter or the inventive 5' regions of the XYL1 or XYL2 gene, respectively. It could be shown, that the expression under control of the XYL1 or XYL2 promoter region may be induced by xylulose, while being repressed by glucose.

### References

Ammerer, G. Expression of genes in yeast using the ADCI promoter. Methods in Enzymology 101:192-201 (1983)
Amore, R., Wilhelm, M. and Hollenberg_{,} C.P. The fermentation of xylose - an analysis of the expression of Bacillus and Actinoplanes xylose isomerase genes in yeast. Appl. Microbiol. Biotechnol. 30:351-357 (1989)
Barnett, J.A. The utilization of sugars by yeasts. In: Advances in carbohydrate chemistry and biochemistry; Tipson, R.S. and Horton, D., eds.; New York: Academic Press, 1976, p. 125-235.
Beggs, J.D. Transformation of yeast by a replicating hybrid plasmid. Nature 275:104-109 (1978)
Blake, M.S., Johnston, K.H., , Russell-Jones, G.J. and Gottschlich, E.C. A rapid, sensitive method for detection of alkaline phosphatase-conjugated anti-antibody on western blots. Anal. Biochem. 136:175-179 (1984)
Bolivar, F., Rodriguez, R.L., Greene, P.J., Betlach, M.C., Heynecker, H.W., Boyer, H.W., Grosa, J.H. and Falkow, S. Construction and characterization of cloning vehicles. II. A multipurpose cloning system. Gene 4:121-136 (1977)
Broach, J.R., Strathern, J.N. and Hicks, J.B. Transformation in yeast: development of a hybrid cloning vector and isolation of the CAN1 gene. Gene 8:121-133 (1979)
Bruinenberg, P.M., VanDijken, J.P. and Scheffers, W.A. An enzymatic analysis of NADPH production and consumption in Candida utilis. J. Gen. Microb. 129:965-971 (1983)
Chan, E.-C., Ueng, P.P. and Chen, L.F. D-xylose fermentation to ethanol by Schizosaccharomyces pombe cloned with xylose isomerase gene. Biotech. Lett. 8:231-234 (1986)
Chan, E.-C., Ueng, P.P. and Chen, L. F. Enviromental effects on D-xylose fermentation by Schizosaccharomyces cerevisiae. Appl. Biotechnol. 20:221-232 (1989)
Ciriacy, M. and Porep, H. Conversion of pentoses to ethanol by baker's yeast. In: Biomolecular Engineering in the European Community, Magnien, E. Dordrecht eds.: Martinus Nijhoff Publishers, 1986, p. 675-681.
Dobson, M.J., Tuite, M.F., Roberts, N.A., Kingsman, A.J., Kingsman, S.M., Perkins, R.E., Conroy, S.C., Dunbar, B. and Fothergill, L.A. Conservation of high efficiency promotor sequences in S. cerevisiae. Nucleic Acid Res. 10:2625-2637 (1982)
Dohmen, R.J., Strasser, A.M.W., Zitomer, R.S. and Hollenberg, C.P. Regulated overproduction of α-amylase by transformation of the amylolytic yeast Schwanniomyces occidentalis. Curr. Genet. 15:319-325 (1989)
Fogel, S. and Welch, J.W.: Tandem gene amplification mediates copper resistance in yeast. Proc. Natl. Acad. Sci. USA 79: 5342-5346 (1982)
Gong, C.S., Chen, L.F., Flickinger, M.C. and Tsao, G.T. Conversion of hemicellulose carbohydrate. Adv. Biochem. Eng. 20:93-118 (1981)
Gubler, U. and Hoffman, B. J. A simple and very efficient method for generating cDNA libraries. Gene 25: 263-269 (1983)
Hagedorn, J. and Ciriacy, M. Isolation and characterisation of xyl mutants in a xylose utilizing yeast, Pichia stipitis. Curr. Genet. 16:27-33 (1989)
Henikoff, S. Unidirectional digestion with exonuclease III creates targeted breakpoints for DNA sequencing. Gene 28:351-359 (1984)
Hohn, B. and Murray, K. Packaging recombinant DNA molecules into bacteriophage particles in vitro. Proc. Natl. Acad. Sci. USA 74:3259-3263 (1977)
Huynh, T.V., Young, R.A. and Davis, R.W. Construction and screening cDNA libraries in 2gt10 and 2gt11. In : DNA cloning, a practical approach, Glover, D., ed.; Oxford: IRL Press, 1985, p. 49-78.
Jeffries, T.W. Utilization of xylose by bacteria, yeast and fungi. Adv. Biochem. Biotech. 27:1-32 (1983)
Jeffries, T.W. Emerging technology for fermenting D-xylose Trends in Biotechnology 3:208-212 (1985)
Johnston, M. and Davis, R.W. Sequences that regulate the divergent GAL1-GAL10 promotor in Saccharomyces cerevisiae. Mol. Cell. Biol. 4:1440-1448 (1984)
Joliff, G., Beguin, P. and Aubert, J.-P. Nucleotide sequence of the cellulase gene celD encoding endoglucanase D of Clostridium thermocellum. Nucleic Acid Res. 14, 8605-8613 (1986)
Kellermann, E. and Hollenberg, C.P. The glucose- and ethanol- dependent regulation of PDC1 from Saccharomyces cerevisiae are controlled by two distinct promotor elements. Curr. Genet. 14:337-344 (1988)
Laemmli, U.K. Cleavage of structural proteins during assembly of the head of bacteriophage T4. Nature 227:680-685 (1970)
Maleszka, R. and Schneider, H. Fermentation of D-xylose, xylitol and D-xylulose by yeasts. Can. J. Microbiol. 28:360-363 (1982)
Maniatis, T., Fritsch, E.F. and Sambrook, E.F. Molecular cloning, a laboratory manual. Cold Spring Harbour, N.Y., Cold Spring Harbour Laboratory (1982)
Myers, A.M., Tzagoloff, A., Kinney, D.M. and Lusty, C.J. Yeast shuttle and integrative vectors with multiple cloning sites suitable for construction of lacZ fusions. Gene 45:299-310 (1986)
N.N. Yeast Genetic Stock Center Cataloge., (1987) 6
Orr-Weaver, T.L., Szostak, J.W. and Rothstein, R.J. Yeast transformation: a model system for the study of recombination. PNAS 78:6354-6358 (1981)
Pacquin, C.E. and Williamson, V.M. Ty insertions account for most of the spontaneus antimycin A resistance mutations during growth at 15^{o}C of Saccharomyces cerevisiae strains lacking ADH1 . Mol. Cell. Biol. 6:70-79 (1986)
Porep, H.J. Xyloseverwertung bei Saccharomyces cerevisiae. University of Duesseldorf, FRG, PhD thesis, (1987)
Russel, D.W., Smith, M., Williamson, V.M. and Young, E.T. Nucleotide sequence of the yeast alcohol dehydrogenase II gene. J. Biol. Chem. 258:2674-2682 (1983)
Russel, D.W., Jensen, R., Zoller, M., Burke, J., Errede, B., Smith, M and Herskowitz, I. : Structure of the Saccharomyces cerevisiae HO gene and analysis of its upstream regulatory region. Mol. Cell. Biol. 6, 4281-4294 (1986)
Sanger, F., Nicklen, S. and Coulson, A.R. DNA sequencing with chain-terminating inhibitors. Proc. Natl. Acad. Sci. USA 74:5463-5467 (1977)
Sartny, A.V., McConaugh, B.L., Lobo, Z., Sundstrom, J.A., Furlong, C.E. and Hall, B.D. Expression of the Escherichia coli xylose isomerase gene in Saccharomyces cerevisiae. Appl. Eur. Microbiol. 53:1996-2000 (1987)
Stinchcomb, D.T., Struhl, K. and Davis, R.W. Isolation and characterization of a yeast chromosomal replicator. Nature 282:39-43 (1979)
Struhl, K., Stinchcomb, D.T., Scherer, S. and Davis, R.W. High frequency transformation of yeast: Autonomous replication of hybrid DNA molecules. Proc. Natl. Acad. Sci. USA 76:1935-1039 (1979)
Teather and Wood Use of Congo red-polysaccharide in enumeration and characterisation of cellulolytic bacteria from bovine rumen. Appl. Env. Microbiol. 43, 777-780 (1982)
Towbin, H., Staelin, T. and Gordon, J. Electrophoretic transfer of proteins from polyacrylamide gels to nitrocellulose sheets: procedure and some applications. Proc. Natl. Acad. Sci USA 76:4350-4354 (1979)
Wu,J.F., Lastick, S.M., Updegraff, D.M. Ethanolproduction from sugars derived from plant biomass by a novel fungus. Nature 321:887-888 (1986)
Zamenhoff, S. Preparation and assay of desoxyribonucleic acids from animal tissue. Methods in Enzymology 3:696-704 (1957)

## Claims (Claims for the following Contracting State(s): AT, BE, CH, LI, DE, DK, FR, GB, GR, IT, LU, NL, SE)

1. DNA sequence, characterized in that said DNA sequence comprises a structural gene coding for a xylose reductase having the following amino acid sequence: 
10
M P S I K L N S G Y
20
D M P A V G F G C W
30
K V D V D T C S E Q
40
I Y R A I K T G Y R
50
L F D G A E D Y A N
60
E K L V G A G V K K
70
A I D E G I V K R E
80
D L F L T S K L W N
90
N Y H H P D N V E K
100
A L N R T L S D L Q
110
V D Y V D L F L I H
120
F P V T F K F V P L
130
E E K Y P P G F Y C
140
G K G D N F D Y E D
 
150
V P I L E T W K A L
160
E K L V K A G K I R
170
S I G V S N F P G A
180
L L L D L L R G A T
190
I K P S V L Q V E H
200
H P Y L Q Q P R L I
210
E F A Q S R G I A V
220
T A Y S S F G P Q S
230
F V E L N Q G R A L
240
N T S P L F E N E T
250
I K A I A A K H G K
260
S P A Q V L L R W S
270
S Q R G I A I I P K
280
S N T V P R L L E N
 
290
K D V N S F D L D E
300
Q D F A D I A K L D
310
I N L R F N D P W D
W D K I P I F V *
 wherein said DNA sequence is capable of expressing said polypeptide in a microorganism.

2. The DNA sequence according to claim 1, characterized in that said DNA sequence further comprises a structural gene coding for xylitol dehydrogenase having the following amino acid sequence:

10
M T A N P S L V L N
20
K I D D I S F E T Y
30
D A P E I S E P T D
40
V L V Q V K K T G I
50
C G S D I H F Y A H
60
G R I G N F V L T K
70
P M V L G H E S A G
80
T V V Q V G K G V T
90
S L K V G D N V A I
100
E P G I P S R F S D
110
E Y K S G H Y N L C
120
P H M A F A A T P N
130
S K E G E P N P P G

140
T L C K Y F K S P E
150
D F L V K L P D H V
160
S L E L G A L V E P
170
L S V G V H A S K L
180
G S V A F G D Y V A
190
V F G A G P V G L L
200
A A A V A K T F G A
210
K G V I V V D I F D
220
N K L K M A K D I G
230
A A T H T F N S K T
240
G G S E E L I K A F
250
G G N V P N V V L E
260
C T G A E P C I K L

270
G V D A I A P G G R
280
F V Q V G N A A G P
290
V S F P I T V F A M
300
K E L T L F G S F R
310
Y G F N D Y K T A V
320
G I F D T N Y Q N G
330
R E N A P I D F E Q
340
L I T H R Y K F K D
350
A I E A Y D L V R A
360
G K G A V K C L I D
G P E *

3. The DNA sequence according to claims 1 or 2, characterized in that said DNA sequence is derived from a yeast, preferably from a yeast selected from a group consisting of the genera Schwanniomyces, Saccharomyces, Kluvyeromyces, Pichia, Hansenula, Candida, Debaryomyces, Metschnikowia, and Pachysolen.

4. The DNA sequence according to claim 3, characterized in that the yeast is Pichia stipitis, preferably Pichia stipitis CBS 5773 (DSM 5855).

5. The DNA sequence according to claim 1, comprising the following nucleotide sequence:

-350
GGATCCACAGACACTAATTGGTTCTA

-310
CATTATTCGTGTTCAGACACAAACCCCAGC

-290
GTTGGCGGTTTCTGTCTGCGTTCCTCCAGC

-250
ACCTTCTTGCTCAACCCCAGAAGGTGCACA

-230
CTGCAGACACACATACATACGAGAACCTGG

-190
AACAAATATCGGTGTCGGTGACCGAAATGT

-170
GCAAACCCAGACACGACTAATAAACCTGGC

-130
AGCTCCAATACCGCCGACAACAGGTGAGGT

-110
GACCGATGGGTGCCAATTAATGTCTGAAA

-70
ATTGGGGTATATAAATATGGCGATTCTCCG

-50
GAGAATTTTTCAGTTTCTTTTCATTTCTC

-10
CAGTATTCTTTTCTATACAACTATACTACA

10 30
ATGCCTTCTATTAAGTTGAACTCTGGTTAC

50
GACATGCCAGCCGTCGGTTTCGGCTGTTGG

70 90
AAAGTCGACGTCGACACCTGTTCTGAACAG

110
ATCTACCGTGCTATCAAGACCGGTTACAGA

130 150
TTGTTCGACGGTGCCGAAGATTACGCCAAC

170
GAAAAGTTAGTTGGTGCCGGTGTCAAGAAG

190 210
GCCATTGACGAAGGTATCGTCAAGCGTGAA

230
GACTTGTTCCTTACCTCCAAGTTGTGGAAC

250 270
AACTACCACCACCCAGACAACGTCGAAAAG

290
GCCTTGAACAGAACCCTTTCTGACTTGCAA

310 330
GTTGACTACGTTGACTTGTTCTTGATCCAC

350
TTCCCAGTCACCTTCAAGTTCGTTCCATTA

370 390
GAAGAAAAGTACCCACCAGGATTCTACTGT

410
GGTAAGGGTGACAACTTCGACTACGAAGAT

430 450
GTTCCAATTTTAGAGACCTGGAAGGCTCTT

470
GAAAAGTTGGTCAAGGCCGGTAAGATCAGA

490 510
TCTATCGGTGTTTCTAACTTCCCAGGTGCT

530
TTGCTCTTGGACTTGTTGAGAGGTGCTACC

550 570
ATCAAGCCATCTGTCTTGCAAGTTGAACAC

590
CACCCATACTTGCAACAACCAAGATTGATC

610 630
GAATTCGCTCAATCCCGTGGTATTGCTGTC

650
ACCGCTTACTCTTCGTTCGGTCCTCAATCT

670 690
TTCGTTGAATTGAACCAAGGTAGAGCTTTG

710
AACACTTCTCCATTGTTCGAGAACGAAACT

730 750
ATCAAGGCTATCGCTGCTAAGCACGGTAAG

770
TCTCCAGCTCAAGTCTTGTTGAGATGGTCT

790 810
TCCCAAAGAGGCATTGCCATCATTCCAAAG

830
TCCAACACTGTCCCAAGATTGTTGGAAAAC

850 870
AAGGACGTCAACAGCTTCGACTTGGACGAA

890
CAAGATTTCGCTGACATTGCCAAGTTGGAC

910 930
ATCAACTTGAGATTCAACGACCCATGGGAC

950
TGGGACAAGATTCCTATCTTCGTCTAAGAA

970 990
GGTTGCTTTATAGAGAGGAAATAAAACCTA

1010
ATATACATTGATTGTACATTTAAAATTGAA

1030 1050
TATTGTAGCTAGCAGATTCGGAAATTTAAA

1070
ATGGGAAGGTGATTCTATCCGTACGAATGA

1090 1110
TCTCTATGTACATACACGTTGAAGATAGCA

1130
GTACAGTAGACATCAAGTCTACAGATCATT

1150 1170
AAACATATCTTAAATTGTAGAAAACTATAA

1190
ACTTTTCAATTCAAACCATGTCTGCCAAGG

1210 1230
AATCAAATGAGATTTTTTTCGCAGCCAAAC

1250
TTGAATCCAAAAATAAAAAACGTCATTGTC

1270 1290
TGAAACAACTCTATCTTATCTTTCACCTCA

1310
TCAATTCATTGCATATCATAAAAGCCTCCG

1330 1350
ATAGCATACAAAACTACTTCTGCATCATAT

1370
CTAAATCATAGTGCCATATTCAGTAACAAT

1390 1410
ACCGGTAAGAAACTTCTATTTTTTTAGTCT

1430
GCCTTAACGAGATGCAGATCGATGCAACGT

1450 1470
AAGATCAAACCCCTCCAGTTGTACAGTCAG

1490
TCATATAGTGAACACCGTACAATATGGTAT

1510 1530
CTACGTTCAAATAGACTCCAATACAGCTGG

1550
TCTGCCCAAGTTGAGCAACTTTAATTTAGA

1570 1590
GACAAAGTCGTCTCTGTTGATGTAGGCACC

1610
ACACATTCTTCTCTTGCCCGTGAACTCTGT

1630 1650
TCTGGAGTGGAAACATCTCCAGTTGTCAAA

1670
TATCAAACACTGACCAGGCTTCAACTGGTA

1690
GAAGATTTCGTTTTCGGATC

6. The DNA sequence according to claim 2, comprising the following nucleotide sequence:

-310                                    -290
TCTAGACCACCCTAAGTCGTCCCTATGTCG

                              -270
TATGTTTGCCTCTACTACAAAGTTACTAGC

-250                                    -230
AAATATCCGCAGCAACAACAGCTGCCCTCT

                              -210
TCCAGCTTCTTAGTGTGTTGGCCGAAAAGG

-190                                    -170
CGCTTTCGGGCTCCAGCTTCTGTCCTCTGC

                              -150
GGCTGCTGCACATAACGCGGGACAATGAC

-130                                    -110
TTCTCCAGCTTTTATTATAAAAGGAGCCAT

                                -90
CTCCTCCAGGTGAAAAATTACGATCAACTT

   -70                                   -50
TTACTCTTTTCCATTGTCTCTTGTGTATAC

                                -30
TCACTTTAGTTTGTTTCAATCACCCCTAAT

   -10                                   10
ACTCTTCACACAATTAAAATGACTGCTAAC

                               30
CCTTCCTTGGTGTTGAACAAGATCGACGAC

   50                                    70
ATTTCGTTCGAAACTTACGATGCCCCAGAA

90
ATCTCTGAACCTACCGATGTCCTCGTCCAG

110 130
GTCAAGAAAACCGGTATCTGTGGTTCCGAC

150
ATCCACTTCTACGCCCATGGTAGAATCGGT

170 190
AACTTCGTTTTGACCAAGCCAATGGTCTTG

210
GGTCACGAATCCGCCGGTACTGTTGTCCAG

230 250
GTTGGTAAGGGTGTCACCTCTCTTAAGGTT

270
GGTGACAACGTCGCTATCGAACCAGGTATT

290 310
CCATCCAGATTCTCCGACGAATACAAGAGC

330
GGTCACTACAACTTGTGTCCTCACATGGCC

350 370
TTCGCCGCTACTCCTAACTCCAAGGAAGGC

390
GAACCAAACCCACCAGGTACCTTATGTAAG

410 430
TACTTCAAGTCGCCAGAAGACTTCTTGGTC

450
AAGTTGCCAGACCACGTCAGCTTGGAACTC

470 490
GGTGCTCTTGTTGAGCCATTGTCTGTTGGT

510
GTCCACGCCTCCAAGTTGGGTTCCGTTGCT

530 550
TTCGGCGACTACGTTGCCGTCTTTGGTGCT

570
GGTCCTGTTGGTCTTTTGGCTGCTGCTGTC

590 610
GCCAAGACCTTCGGTGCTAAGGGTGTCATC

630
GTCGTTGACATTTTCGACAACAAGTTGAAG

650 670
ATGGCCAAGGACATTGGTGCTGCTACTCAC

690
ACCTTCAACTCCAAGACCGGTGGTTCTGAA

710 730
GAATTGATCAAGGCTTTCGGTGGTAACGTG

750
CCAAACGTCGTTTTGGAATGTACTGGTGCT

770 790
GAACCTTGTATCAAGTTGGGTGTTGACGCC

810
ATTGCCCCAGGTGGTCGTTTCGTTCAAGTT

830 850
GGTAACGCTGCTGGTCCAGTCAGCTTCCCA

870
ATCACCGTTTTCGCCATGAAGGAATTGACT

890 910
TTGTTCGGTTCTTTCAGATACGGATTCAAC

930
GACTACAAGACTGCTGTTGGAATCTTTGAC

950 970
ACTAACTACCAAAACGGTAGAGAAAATGCT

990
CCAATTGACTTTGAACAATTGATCACCCAC

1010 1030
AGATACAAGTTCAAGGACGCTATTGAAGCC

1050
TACGACTTGGTCAGAGCCGGTAAGGGTGCT

1070 1090
GTCAAGTGTCTCATTGACGGCCCTGAGTAA

1110
GTCAACCGCTTGGCTGGCCCAAAGTGAACC

1130 1150
AGAAACGAAAATGATTATCAAATAGCTTTA

1170
TAGACCTTTATCGAAATTTATGTAAACTAA

1190 1210
TAGAAAAGACAGTGTAGAAGTTATATGGTT

1230
GCATCACGTGAGTTTCTTGAATTCTTGAAA

1250                                    1270
GTGAAGTCTTGGTCGGAACAAACAAACAAA

                    1290
AAAATATTTTCAGCAAGAGTTGATTTCTTT

1310                                    1330
TCTGGAGATTTTGGTAATTGACAGAGAACC

                    1350
CCTTTCTGCTATTGCCATCTAAACATCCTT

1370                                    1390
GAATAGAACTTTACTGGATGGCCGCCTAGT

                    1410
GTTGAGTATATATTATCAACCAAAATCCTG

1430                                    1450
TATATAGTCTCTGAAAAATTTGACTATCCT

                    1470
AACTTAACAAAAGAGCACCATAATGCAAGC

1490                                    1510
TCATAGTTCTTAGAGACACCAACTATACTT

                    1530
AGCCAAACAAAATGTCCTTGGCCTCTAAAG

1550                                    1570
AAGCATTCAGCAAGCTTCCCCAGAAGTTGC

                    1590
ACAACTTCTTCATCAAGTTTACCCCCAGAC

1610                                    1630
CGTTTGCCGAATATTCGGAAAAGCCTTCGA

CTATAGTGGATCC

7. The DNA sequence according to any of claims 1 to 6, characterized in that it is obtained by recombinant DNA technology from natural and/or cDNA and/or chemically synthesised DNA.

8. The combination of DNA sequences, characterized in that said combination comprises a first DNA sequence according to any of claims 1 to 7 and one or more further DNA sequences capable of regulating the expression of a structural gene encoded by said DNA sequence in a host microorganism.

9. The combination of DNA sequences according to claim 8, characterized in that said combination comprises modifications of the DNA sequences retaining their capability to express a functional enzyme having xylose reductase or xylitol dehydrogenase activity.

10. The combination of DNA sequences according to claim 8 or 9, characterized in that said structural gene contains DNA sequences derived from the structural gene coding for xylose reductase or xylitol dehydrogenase which modify said protein product while retaining its functions in such a way that said protein product is expressed as a gene product having enzymatic activity.

11. The combination of DNA sequences according to any of claims 8 to 10, characterized in that said DNA sequences capable of regulating the expression of said structural gene in a host microorganism are derived from said host microorganism.

12. The combination according to claim 11, characterized in that said DNA sequences capable of regulating the expression are inducible promoters.

13. The combination according to claim 12, characterized in that said DNA sequences capable of regulating the expression are selected from the following promoters:
ADH1, ADH2, PDC, GAL1/10.

14. The combination according to any of claims 11 to 13, characterized in that said DNA sequence capable of regulating the expression of said structural gene is a strong promoter, leading to over expression of the protein encoded by said structural gene.

15. A vector, characterized in that said vector comprises a DNA sequence according to any of claims 1 to 7 or a combination of DNA sequences according to any of claims 8 to 14.

16. The vector according to claim 15, characterized in that said vector is selected from the group comprising the plasmids pR1, pR2, pD1, pD2, pRD1, pXRa, pXRb, pXDH, pXR, pXDH-HIS3, pXR-LEU2.

17. A microorganism, characterized in that said microorganism is capable of expressing a xylose reductase or xylose reductase and xylitol dehydrogenase as a result of having received DNA sequences comprising the DNA sequences according to any of claims 1 to 7 or a combination of DNA sequences according to any of claims 8 to 14, coding for said xylose reductase or said xylose reductase and said xylitol dehydrogenase, by recombinant DNA technology.

18. The microorganism according to claim 17, characterized in that said microorganism is selected from a group consisting of yeast of the genera Saccharomyces, Schizosaccharomyces, Schwanniomyces, Kluyveromyces, Pichia, Hansenula, Candida, Debaryomyces, Metschnikowia, Pachysolen, or Paecilomyces or bacteria of the genus Zymomonas.

19. The microorganism according to claim 18, characterized in that said microorganism is Saccharomyces cerevisiae.

20. The microorganism according to claim 18, characterized in that said microorganism is Schizosaccharomyces pombe.

21. The microorganism according to any of claims 17 to 20, characterized in that said DNA sequence or combination of DNA sequences is integrated into the genome of said microorganism.

22. The microorganism according to any of claims 17 to 21, characterized in that said microorganism is useful in biomass production, in food industry and fermentation processes.

23. The microorganism according to claim 22, characterized in that said microorganism is useful for fermentation of xylose into ethanol.

24. A method for producing xylose reductase or xylose reductase and xylitol dehydrogenase by cultivating a microorganism according to any of claims 17 to 21 under suitable conditions and recovering said enzyme(s) in a manner known per se.

25. The method according to claim 24, characterized in that said microorganism is selected for efficient fermentation of xylulose.

26. The method according to claim 24 or 25, characterized in that said microorganism has received said DNA sequences or said combination of DNA sequences by transformation using a vector, said vector being preferably a DNA fragment or a plasmid.

27. The method according to claim 26, characterized in that said vector contains DNA, which is homologous to DNA of said microorganism, leading to integration into the genome of said microorganism.

28. An ethanol manufacturing process, characterized in that a microorganism according to any of claims 17 to 23 is used.

29. A process according to claim 28, characterized in that the fermentation process is adapted for the production of alcoholic beverages or single cell protein produced from substrates containing free xylose, preferably released by xylanase and/or xylosidase activity.

30. A process for production of biomass, characterized in that a host microorganism according to any of claims 17 to 23 is used.

## Claims (Claims for the following Contracting State(s): ES)

1. A method for preparing a DNA sequence, which DNA sequence comprises a structural gene coding for a xylose reductase having the following amino acid sequence: 
10
M P S I K L N S G Y

20
D M P A V G F G C W

30
K V D V D T C S E Q

40
I Y R A I K T G Y R

50
L F D G A E D Y A N

60
E K L V G A G V K K

70
A I D E G I V K R E

80
D L F L T S K L W N

90
N Y H H P D N V E K

100
A L N R T L S D L Q

110
V D Y V D L F L I H

120
F P V T F K F V P L

130
E E K Y P P G F Y C

140
G K G D N F D Y E D
 
150
V P I L E T W K A L
160
E K L V K A G K I R
170
S I G V S N F P G A
180
L L L D L L R G A T
190
I K P S V L Q V E H
200
H P Y L Q Q P R L I
210
E F A Q S R G I A V
220
T A Y S S F G P Q S
230
F V E L N Q G R A L
240
N T S P L F E N E T
250
I K A I A A K H G K
260
S P A Q V L L R W S
270
S Q R G I A I I P K
280
S N T V P R L L E N
 
290
K D V N S F D L D E
300
Q D F A D I A K L D
310
I N L R F N D P W D
W D K I P I F V *
 said DNA sequence being capable of expressing said polypeptide in a microorganism, wherein said DNA sequence is prepared by recombinant DNA technology from natural and/or cDNA and/or chemically synthesized DNA.

2. A method according to claim 1, wherein said DNA sequence further comprises a structural gene encoding xylitol dehydrogenase having the following amino acid sequence:

10
M T A N P S L V L N
20
K I D D I S F E T Y
30
D A P E I S E P T D
40
V L V Q V K K T G I
50
C G S D I H F Y A H
60
G R I G N F V L T K
70
P M V L G H E S A G
80
T V V Q V G K G V T
90
S L K V G D N V A I
100
E P G I P S R F S D
110
E Y K S G H Y N L C
120
P H M A F A A T P N
130
S K E G E P N P P G

140
T L C K Y F K S P E

150
D F L V K L P D H V

160
S L E L G A L V E P

170
L S V G V H A S K L

180
G S V A F G D Y V A

190
V F G A G P V G L L

200
A A A V A K T F G A

210
K G V I V V D I F D

220
N K L K M A K D I G

230
A A T H T F N S K T

240
G G S E E L I K A F

250
G G N V P N V V L E

260
C T G A E P C I K L

270
G V D A I A P G G R
280
F V Q V G N A A G P
290
V S F P I T V F A M
300
K E L T L F G S F R
310
Y G F N D Y K T A V
320
G I F D T N Y Q N G
330
R E N A P I D F E Q
340
L I T H R Y K F K D
350
A I E A Y D L V R A
360
G K G A V K C L I D
G P E *

3. The method according to any of claims 1 or 2, characterized in that said DNA sequence is derived from a yeast, preferably from a yeast selected from a group consisting of the genera Schwanniomyces, Saccharomyces, Kluyveromyces, Pichia, Hansenula, Candida, Debaryomyces, Metschnikowia, and Pachysolen.

4. The method according to claim 3, characterized in that the yeast is Pichia stipitis, preferably Pichia stipitis 5773 (DSM 5855).

5. The method according to claim 1, wherein the DNA sequence comprises the following nucleotide sequence:

-350
GGATCCACAGACACTAATTGGTTCTA

-310
CATTATTCGTGTTCAGACACAAACCCCAGC

-290
GTTGGCGGTTTCTGTCTGCGTTCCTCCAGC

-250
ACCTTCTTGCTCAACCCCAGAAGGTGCACA

-230
CTGCAGACACACATACATACGAGAACCTGG

-190
AACAAATATCGGTGTCGGTGACCGAAATGT

-170
GCAAACCCAGACACGACTAATAAACCTGGC

-130
AGCTCCAATACCGCCGACAACAGGTGAGGT

-110
GACCGATGGGGTGCCAATTAATGTCTGAAA

-70
ATTGGGGTATATAAATATGGCGATTCTCCG

-50
GAGAATTTTTCAGTTTTCTTTTCATTTCTC

-10
CAGTATTCTTTTCTATACAACTATACTACA

10 30
ATGCCTTCTATTAAGTTGAACTCTGGTTAC

50
GACATGCCAGCCGTCGGTTTCGGCTGTTGG

70 90
AAAGTCGACGTCGACACCTGTTCTGAACAG

110
ATCTACCGTGCTATCAAGACCGGTTACAGA

130 150
TTGTTCGACGGTGCCGAAGATTACGCCAAC

170
GAAAAGTTAGTTGGTGCCGGTGTCAAGAAG

190 210
GCCATTGACGAAGGTATCGTCAAGCGTGAA

230
GACTTGTTCCTTACCTCCAAGTTGTGGAAC

250 270
AACTACCACCACCCAGACAACGTCGAAAAG

290
GCCTTGAACAGAACCCTTTCTGACTTGCAA

310 330
GTTGACTACGTTGACTTGTTCTTGATCCAC

350
TTCCCAGTCACCTTCAAGTTCGTTCCATTA

370 390
GAAGAAAAGTACCCACCAGGATTCTACTGT

410
GGTAAGGGTGACAACTTCGACTACGAAGAT

430 450
GTTCCAATTTTAGAGACCTGGAAGGCTCTT

470
GAAAAGTTGGTCAAGGCCGGTAAGATCAGA

490 510
TCTATCGGTGTTTCTAACTTCCCAGGTGCT

530
TTGCTCTTGGACTTGTTGAGAGGTGCTACC

550 570
ATCAAGCCATCTGTCTTGCAAGTTGAACAC

590
CACCCATACTTGCAACAACCAAGATTGATC

610 630
GAATTCGCTCAATCCCGTGGTATTGCTGTC

650
ACCGCTTACTCTTCGTTCGGTCCTCAATCT

670 690
TTCGTTGAATTGAACCAAGGTAGAGCTTTG

710
AACACTTCTCCATTGTTCGAGAACGAAACT

730 750
ATCAAGGCTATCGCTGCTAAGCACGGTAAG

770
TCTCCAGCTCAAGTCTTGTTGAGATGGTCT

790 810
TCCCAAAGAGGCATTGCCATCATTCCAAAG

830
TCCAACACTGTCCCAAGATTGTTGGAAAAC

850 870
AAGGACGTCAACAGCTTCGACTTGGACGAA

890
CAAGATTTCGCTGACATTGCCAAGTTGGAC

910 930
ATCAACTTGAGATTCAACGACCCATGGGAC

950
TGGGACAAGATTCCTATCTTCGTCTAAGAA

970 990
GGTTGCTTTATAGAGAGGAAATAAAACCTA

1010
ATATACATTGATTGTACATTTAAAATTGAA

1030 1050
TATTGTAGCTAGCAGATTCGGAAATTTAAA

1070
ATGGAAGGTGATTCTATCCGTACGAATGA

1090 1110
TCTCTATGTACATACACGTTGAAGATAGCA

1130
GTACAGTAGACATCAAGTCTACAGATCATT

1150 1170
AAACATATCTTAAATTGTAGAAAACTATAA

1190
ACTTTTCAATTCAAACCATGTCTGCCAAGG

1210 1230
AATCAAATGAGATTTTTTCGCAGCCAAAC

1250
TTGAATCCAAAAATAAAAACGTCATTGTC

1270 1290
TGAAACAACTCTATCTTATCTTTCACCTCA

1310
TCAATTCATTGCATATCATAAAGCCTCCG

1330                                    1350
ATAGCATACAAAACTACTTCTGCATCATAT

                    1370
CTAAATCATAGTGCCATATTCAGTAACAAT

1390                                    1410
ACCGGTAAGAAACTTCTATTTTTTTAGTCT

                    1430
GCCTTAACGAGATGCAGATCGATGCAACGT

1450                                    1470
AAGATCAAACCCCTCCAGTTGTACAGTCAG

                    1490
TCATATAGTGAACACCGTACAATATGGTAT

1510                                    1530
CTACGTTCAAATAGACTCCAATACAGCTGG

                    1550
TCTGCCCAAGTTGAGCAACTTTAATTTAGA

1570                                    1590
GACAAAGTCGTCTCTGTTGATGTAGGCACC

                    1610
ACACATTCTTCTCTTGCCCGTGAACTCTGT

1630                                    1650
TCTGGAGTGGAAACATCTCCAGTTGTCAAA

                    1670
TATCAAACACTGACCAGGCTTCAACTGGTA

1690
GAAGATTTCGTTTTCGGATC

6. The method according to claim 2, wherein the DNA sequence comprises the following nucleotide sequence:

-310 -290
TCTAGACCACCCTAAGTCGTCCCTATGTCG

-270
TATGTTTGCCTCTACTACAAAGTTACTAGC

-250 -230
AAATATCCGCAGCAACAACAGCTGCCCTCT

-210
TCCAGCTTCTTAGTGTGTTGGCCGAAAAGG

-190 -170
CGCTTTCGGGCTCCAGCTTCTGTCCTCTGC

-150
GGCTGCTGCACATAACGCGGGACAATGAC

-130 -110
TTCTCCAGCTTTTATTATAAAGGAGCCAT

-90
CTCCTCCAGGTGAAAAATTACGATCAACTT

-70 -50
TTACTCTTTTCCATTGTCTCTTGTGTATAC

-30
TCACTTTAGTTTGTTTCAATCACCCCTAAT

-10 10
ACTCTTCACACAATTAAAATGACTGCTAAC

30
CCTTCCTTGGTGTTGAACAAGATCGACGAC

50 70
ATTTCGTTCGAAACTTACGATGCCCCAGAA

90
ATCTCTGAACCTACCGATGTCCTCGTCCAG

110 130
GTCAAGAAAACCGGTATCTGTGGTTCCGAC

150
ATCCACTTCTACGCCCATGGTAGAATCGGT

170 190
AACTTCGTTTTGACCAAGCCAATGGTCTTG

210
GGTCACGAATCCGCCGGTACTGTTGTCCAG

230 250
GTTGGTAAGGGTGTCACCTCTCTTAAGGTT

270
GGTGACAACGTCGCTATCGAACCAGGTATT

290 310
CCATCCAGATTCTCCGACGAATACAAGAGC

330
GGTCACTACAACTTGTGTCCTCACATGGCC

350 370
TTCGCCGCTACTCCTAACTCCAAGGAAGGC

390
GAACCAAACCCACCAGGTACCTTATGTAAG

410 430
TACTTCAAGTCGCCAGAAGACTTCTTGGTC

450
AAGTTGCCAGACCACGTCAGCTTGGAACTC

470 490
GGTGCTCTTGTTGAGCCATTGTCTGTTGGT

510
GTCCACGCCTCCAAGTTGGGTTCCGTTGCT

530 550
TTCGGCGACTACGTTGCCGTCTTTGGTGCT

570
GGTCCTGTTGGTCTTTTGGCTGCTGCTGTC

590 610
GCCAAGACCTTCGGTGCTAAGGGTGTCATC

630
GTCGTTGACATTTTCGACAACAAGTTGAAG

650 670
ATGGCCAAGGACATTGGTGCTGCTACTCAC

690
ACCTTCAACTCCAAGACCGGTGGTTCTGAA

710 730
GAATTGATCAAGGCTTTCGGTGGTAACGTG

750
CCAAACGTCGTTTTGGAATGTACTGGTGCT

770 790
GAACCTTGTATCAAGTTGGGTGTTGACGCC

810
ATTGCCCCAGGTGGTCGTTTCGTTCAAGTT

830 850
GGTAACGCTGCTGGTCCAGTCAGCTTCCCA

870
ATCACCGTTTTCGCCATGAAGGAATTGACT

890 910
TTGTTCGGTTCTTTCAGATACGGATTCAAC

930
GACTACAAGACTGCTGTTGGAATCTTTGAC

950 970
ACTAACTACCAAAACGGTAGAGAAAATGCT

990
CCAATTGACTTTGAACAATTGATCACCCAC

1010 1030
AGATACAAGTTCAAGGACGCTATTGAAGCC

1050
TACGACTTGGTCAGAGCCGGTAAGGGTGCT

1070 1090
GTCAAGTGTCTCATTGACGGCCCTGAGTAA

1110
GTCAACCGCTTGGCTGGCCCAAAGTGAACC

1130 1150
AGAAACGAAAATGATTATCAAATAGCTTTA

1170
TAGACCTTTATCGAAATTTATGTAAACTAA

1190 1210
TAGAAAAGACAGTGTAGAAGTTATATGGTT

1230
GCATCACGTGAGTTTCTTGAATTCTTGAAA

1250                                    1270
GTGAAGTCTTGGTCGGAACAAACAAACAAA

                    1290
AAAATATTTTCAGCAAGAGTTGATTTCTTT

1310                                    1330
TCTGGAGATTTTGGTAATTGACAGAGAACC

                    1350
CCTTTCTGCTATTGCCATCTAAACATCCTT

1370                                    1390
GAATAGAACTTTACTGGATGGCCGCCTAGT

                    1410
GTTGAGTATATATTATCAACCAAAATCCTG

1430                                    1450
TATATAGTCTCTGAAAAATTTGACTATCCT

                    1470
AACTTAACAAAAGAGCACCATAATGCAAGC

1490                                    1510
TCATAGTTCTTAGAGACACCAACTATACTT

                    1530
AGCCAAACAAAATGTCCTTGGCCTCTAAAG

1550                                    1570
AAGCATTCAGCAAGCTTCCCCAGAAGTTGC

                    1590
ACAACTTCTTCATCAAGTTTACCCCCAGAC

1610                                    1630
CGTTTGCCGAATATTCGGAAAAGCCTTCGA

CTATAGTGGATCC

7. A method for preparing a combination of DNA sequences, said method comprising combining a first DNA sequence obtainable according to any of claims 1 to 6 and one or more further DNA sequences capable of regulating the expression of a structural gene encoded by said DNA sequence in a host microorganism in a manner known per se.

8. The method according to claim 7, wherein said combination of sequences comprises modifications of the DNA sequences retaining their capability to express a functional enzyme having xylose reductase or xylitol dehydrogenase activity.

9. The method according to any of claims 7 or 8, wherein said structural gene contains DNA sequences derived from the structural gene coding for xylose reductase or xylitol dehydrogenase which modify said protein product while retaining its functions in such a way that said protein product is expressed as a gene product having enzymatic activity.

10. The method according to any of claims 7 to 9, wherein said DNA sequences capable of regulating the expression of said structural gene in a host microorganism are derived from said host microorganism.

11. The method according to claim 10, wherein said DNA sequences capable of regulating the expression are inducible promoters.

12. The method according to claim 11, characterized in that said DNA sequences capable of regulating the expression are selected from the following promoters:
ADH1, ADH2, PDC, GAL1/10.

13. The method according to any of claims 10 to 12, wherein said DNA sequences capable of regulating the expression of said structural gene is a strong promoter, leading to over expression of the protein encoded by said structural gene.

14. The method for preparing a vector, said method comprising inserting a DNA sequence obtainable according to any of claims 1 to 6 or a combination of DNA sequences obtainable according to any of claims 7 to 13 into a host plasmid.

15. The method according to claim 14, characterized in that it produces a vector selected from the group comprising the plasmids pR1, pR2, pD1, pD2, pRD1, pXRa, pXRb, pXDH, pXR, pXDH-HIS3, pXR-LEU2.

16. A method for preparing a microorganism being capable of expressing a xylose reductase or xylose reductase and xylitol dehydrogenase, wherein DNA sequences comprising the DNA sequences obtainable according to any of claims 1 to 6 or a combination of DNA sequences obtainable according to any of claims 7 to 13, coding for said xylose reductase or said xylose reductase and said xylitol dehydrogenase, are introduced into a host microorganism.

17. The method according to claim 16, characterized in that said host microorganism is selected from a group consisting of yeast of the genera Saccharomyces, Schizosaccharomyces, Schwanniomyces, Kluyveromyces, Pichia, Hansenula, Candida, Debaryomyces, Metschnikowia, Pachysolen, or Paecilomyces or bacteria of the genus Zymomonas.

18. The method according to claim 17, characterized in that said microorganism is Saccharomyces cerevisiae.

19. The method according to claim 17, characterized in that said microorganism is Schizosaccharomyces pombe.

20. The method according to any of claims 16 to 19, characterized in that said DNA sequence or combination of DNA sequences is integrated into the genome of said microorganism.

21. The method according to any of claims 17 to 21, characterized in that said microorganism is useful in biomass production, in food industry and fermentation processes.

22. The method according to claim 21, characterized in that said microoganism is useful for fermentation of xylose into ethanol.

23. A method for producing xylose reductase or xylose reductase and xylitol dehydrogenase by cultivating a microorganism obtainable according to any of claims 16 to 20 under suitable conditions and recovering said enzyme(s) in a manner known per se.

24. The method according to claim 23, characterized in that said microorganism is selected for efficient fermentation of xylulose.

25. The method according to claim 23 or 24, characterized in that said microoganism has received said DNA sequences or said combination of DNA sequences by transformation using a vector, said vector being preferably a DNA fragment or a plasmid.

26. The method according to claim 25, characterized in that said vector contains DNA, which is homologous to DNA of said microorganism, leading to integration into the genome of said microorganism.

27. An ethanol manufacturing process, characterized in that a microorganism obtainable according to any of claims 16 to 22 is used.

28. A process according to claim 27, characterized in that the fermentation process is adapted for the production of alcoholic beverages or single cell protein produced from substrates containing free xylose, preferably released by xylanase and/or xylosidase activity.

29. A process for production of biomass, characterized in that a host microorganism according to any of claims 16 to 22 is used.

## Patentansprüche (Patentansprüche für folgende(n) Vertragsstaat(en): AT, BE, CH, LI, DE, DK, FR, GB, GR, IT, LU, NL, SE)

1. DNA-Sequenz, dadurch gekennzeichnet, daß die DNA-Sequenz ein Strukturgen umfaßt, das für eine Xylosereduktase mit der folgenden Aminosäuresequenz kodiert: 
10
M P S I K L N S G Y
20
D M P A V G F G C W
30
K V D V D T C S E Q
40
I Y R A I K T G Y R
50
L F D G A E D Y A N
60
E K L V G A G V K K
70
A I D E G I V K R E
80
D L F L T S K L W N
90
N Y H H P D N V E K
100
A L N R T L S D L Q
110
V D Y V D L F L I H
120
F P V T F K F V P L
130
E E K Y P P G F Y C
140
G K G D N F D Y E D
 
150
V P I L E T W K A L
160
E K L V K A G K I R
170
S I G V S N F P G A
180
L L L D L L R G A T
190
I K P S V L Q V E H
200
H P Y L Q Q P R L I
210
E F A Q S R G I A V
220
T A Y S S F G P Q S
230
F V E L N Q G R A L
240
N T S P L F E N E T
250
I K A I A A K H G K
260
S P A Q V L L R W S
270
S Q R G I A I I P K
280
S N T V P R L L E N
 
290
K D V N S F D L D E
300
Q D F A D I A K L D
310
I N L R F N D P W D
W D K I P I F V *
 wobei die DNA-Sequenz das Polypeptid in einem Mikroorganismus exprimieren kann.

2. DNA-Sequenz nach Anspruch 1, dadurch gekennzeichnet, daß die DNA-Sequenz weiter ein Strukturgen umfaßt, das für eine Xylitoldehydrogenase mit der folgenden Aminosäuresequenz kodiert:

10
M T A N P S L V L N
20
K I D D I S F E T Y
30
D A P E I S E P T D
40
V L V Q V K K T G I
50
C G S D I H F Y A H
60
G R I G N F V L T K
70
P M V L G H E S A G
80
T V V Q V G K G V T
90
S L K V G D N V A I
100
E P G I P S R F S D
110
E Y K S G H Y N L C
120
P H M A F A A T P N
130
S K E G E P N P P G

140
T L C K Y F K S P E
150
D F L V K L P D H V
160
S L E L G A L V E P
170
L S V G V H A S K L
180
G S V A F G D Y V A
190
V F G A G P V G L L
200
A A A V A K T F G A
210
K G V I V V D I F D
220
N K L K M A K D I G
230
A A T H T F N S K T
240
G G S E E L I K A F
250
G G N V P N V V L E
260
C T G A E P C I K L

270
G V D A I A P G G R
280
F V Q V G N A A G P
290
V S F P I T V F A M
300
K E L T L F G S F R
310
Y G F N D Y K T A V
320
G I F D T N Y Q N G
330
R E N A P I D F E Q
340
L I T H R Y K F K D
350
A I E A Y D L V R A
360
G K G A V K C L I D
G P E *

3. DNA-Sequenz nach Anspruch 1 oder 2, dadurch gekennzeichnet, daß die DNA-Sequenz von einer Hefe abgeleitet ist, bevorzugt von einer Hefe, die aus der aus den Gattungen Schwanniomyces, Saccharomyces, Kluyeromyces, Pichia, Hansenula, Candida, Debaryomyces, Metschnikowia und Pachysolen bestehenden Gruppe ausgewählt ist.

4. DNA-Sequenz nach Anspruch 3, dadurch gekennzeichnet, daß die Hefe Pichia stipitis ist, bevorzugt Pichia stipitis CBS 5773 (DSM 5855).

5. DNA-Sequenz nach Anspruch 1, umfassend die folgende Nukleotidsequenz:

-350
GGATCCACAGACACTAATTGGTTCTA

-310
CATTATTCGTGTTCAGACACAAACCCCAGC

-290
GTTGGCGGTTTCTGTCTGCGTTCCTCCAGC

-250
ACCTTCTTGCTCAACCCCAGAAGGTGCACA

-230
CTGCAGACACACATACATACGAGAACCTGG

-190
AACAAATATCGGTGTCGGTGACCGAAATGT

-170
GCAAACCCAGACACGACTAATAAACCTGGC

-130
AGCTCCAATACCGCCGACAACAGGTGAGGT

-110
GACCGATGGGGTGCCAATTAATGTCTGAAA

-70
ATTGGGGTATATAAATATGGCGATTCTCCG

-50
GAGAATTTTTCAGTTTTCTTTTCATTTCTC

-10
CAGTATTCTTTTCTATACAACTATACTACA

10 30
ATGCCTTCTATTAAGTTGAACTCTGGTTAC

50
GACATGCCAGCCGTCGGTTTCGGCTGTTGG

70 90
AAAGTCGACGTCGACACCTGTTCTGAACAG

110
ATCTACCGTGCTATCAAGACCGGTTACAGA

130 150
TTGTTCGACGGTGCCGAAGATTACGCCAAC

170
GAAAAGTTAGTTGGTGCCGGTGTCAAGAAG

190 210
GCCATTGACGAAGGTATCGTCAAGCGTGAA

230
GACTTGTTCCTTACCTCCAAGTTGTGGAAC

250 270
AACTACCACCACCCAGACAACGTCGAAAAG

290
GCCTTGAACAGAACCCTTTCTGACTTGCAA

310 330
GTTGACTACGTTGACTTGTTCTTGATCCAC

350
TTCCCAGTCACCTTCAAGTTCGTTCCATTA

370 390
GAAGAAAAGTACCCACCAGGATTCTACTGT

410
GGTAAGGGTGACAACTTCGACTACGAAGAT

430 450
GTTCCAATTTTAGAGACCTGGAAGGCTCTT

470
GAAAAGTTGGTCAAGGCCGGTAAGATCAGA

490 510
TCTATCGGTGTTTCTAACTTCCCAGGTGCT

530
TTGCTCTTGGACTTGTTGAGAGGTGCTACC

550 570
ATCAAGCCATCTGTCTTGCAAGTTGAACAC

590
CACCCATACTTGCAACAACCAAGATTGATC

610 630
GAATTCGCTCAATCCCGTGGTATTGCTGTC

650
ACCGCTTACTCTTCGTTCGGTCCTCAATCT

670 690
TTCGTTGAATTGAACCAAGGTAGAGCTTTG

710
AACACTTCTCCATTGTTCGAGAACGAAACT

730 750
ATCAAGGCTATCGCTGGTAAGCACGGTAAG

770
TCTCCAGCTCAAGTCTTGTTGAGATGGTCT

790 810
TCCCAAAGAGGCATTGCCATCATTCCAAAG

830
TCCAACACTGTCCCAAGATTGTTGGAAAAC

850 870
AAGGACGTCAACAGCTTCGACTTGGACGAA

890
CAAGATTTCGCTGACATTGCCAAGTTGGAC

910 930
ATCAACTTGAGATTCAACGACCCATGGGAC

950
TGGGACAAGATTCCTATCTTCGTCTAAGAA

970 990
GGTTGCTTTATAGAGAGGAAATAAAACCTA

1010
ATATACATTGATTGTACATTTAAAATTGAA

1030 1050
TATTGTAGCTAGCAGATTCGGAAATTTAAA

1070
ATGGAAGGTGATTCTATCCGTACGAATGA

1090 1110
TCTCTATGTACATACACGTTGAAGATAGCA

1130
GTACAGTAGACATCAAGTCTACAGATCATT

1150 1170
AAACATATCTTAAATTGTAGAAAACTATAA

1190
ACTTTTCAATTCAAACCATGTCTGCCAAGG

1210 1230
AATCAAATGAGATTTTTTCGCAGCCAAAC

1250
TTGAATCCAAAAATAAAAAACGTCATTGTC

1270 1290
TGAAACAACTCTATCTTATCTTTCACCTCA

1310
TCAATTCATTGCATATCATAAAAGCCTCCG

1330 1350
ATAGCATACAAAACTACTTCTGCATCATAT

1370
CTAAATCATAGTGCCATATTCAGTAACAAT

1390 1410
ACCGGTAAGAAACTTCTATTTTTTTAGTCT

1430
GCCTTAACGAGATGCAGATCGATGCAACGT

1450 1470
AAGATCAAACCCCTCCAGTTGTACAGTCAG

1490
TCATATAGTGAACACCGTACAATATGGTAT

1510 1530
CTACGTTCAAATAGACTCCAATACAGCTGG

1550
TCTGCCCAAGTTGAGCAACTTTAATTTAGA

1570 1590
GACAAAGTCGTCTCTGTTGATGTAGGCACC

1610
ACACATTCTTCTCTTGCCCGTGAACTCTGT

1630 1650
TCTGGAGTGGAAACATCTCCAGTTGTCAAA

1670
TATCAAACACTGACCAGGCTTCAACTGGTA

1690
GAAGATTTCGTTTTCGGGATC

6. DNA-Sequenz nach Anspruch 2, umfassend die folgende Nukleotidsequenz:

-310 -290
TCTAGACCACCCTAAGTCGTCCCTATGTCG
-270
TATGTTTGCCTCTACTACAAAGTTACTAGC
-250 -230
AAATATCCGCAGCAACAACAGCTGCCCTCT
-210
TCCAGCTTCTTAGTGTGTTGGCCGAAAAGG
-190 -170
CGCTTTCGGGCTCCAGCTTCTGTCCTCTGC
-150
GGCTGCTGCACATAACGCGGGACAATGAC
-130 -110
TTCTCCAGCTTTTATTATAAAAGGAGCCAT
-90
CTCCTCCAGGTGAAAAATTACGATCAACTT
-70 -50
TTACTCTTTTCCATTGTCTCTTGTGTATAC
-30
TCACTTTAGTTTGTTTCAATCACCCCTAAT
-10 10
ACTCTTCACACAATTAAAATGACTGCTAAC
30
CCTTCCTTGGTGTTGAACAAGATCGACGAC
50 70
ATTTCGTTCGAAACTTACGATGCCCCAGAA

90
ATCTCTGAACCTACCGATGTCCTCGTCCAG

110 130
GTCAAGAAAACCGGTATCTGTGGTTCCGAC

150
ATCCACTTCTACGCCCATGGTAGAATCGGT

170 190
AACTTCGTTTTGACCAAGCCAATGGTCTTG

210
GGTCACGAATCCGCCGGTACTGTTGTCCAG

230 250
GTTGGTAAGGGTGTCACCTCTCTTAAGGTT

270
GGTGACAACGTCGCTATCGAACCAGGTATT

290 310
CCATCCAGATTCTCCGACGAATACAAGAGC

330
GGTCACTACAACTTGTGTCCTCACATGGCC

350 370
TTCGCCGCTACTCCTAACTCCAAGGAAGGC

390
GAACCAAACCCACCAGGTACCTTATGTAAG

410 430
TACTTCAAGTCGCCAGAAGACTTCTTGGTC

450
AAGTTGCCAGACCACGTCAGCTTGGAACTC

470 490
GGTGCTCTTGTTGAGCCATTGTCTGTTGGT

510
GTCCACGCCTCCAAGTTGGGTTCCGTTGCT

530 550
TTCGGCGACTACGTTGCCGTCTTTGGTGCT

570
GGTCCTGTTGGTCTTTTGGCTGCTGCTGTC

590 610
GCCAAGACCTTCGGTGCTAAGGGTGTCATC

630
GTCGTTGACATTTTCGACAACAAGTTGAAG

650 670
ATGGCCAAGGACATTGGTGCTGCTACTCAC

690
ACCTTCAACTCCAAGACCGGTGGTTCTGAA

710 730
GAATTGATCAAGGCTTTCGGTGGTAACGTG

750
CCAAACGTCGTTTTGGAATGTACTGGTGCT

770 790
GAACCTTGTATCAAGTTGGGTGTTGACGCC

810
ATTGCCCCAGGTGGTCGTTTCGTTCAAGTT

830 850
GGTAACGCTGCTGGTCCAGTCAGCTTCCCA

870
ATCACCGTTTTCGCCATGAAGGAATTGACT

890 910
TTGTTCGGTTCTTTCAGATACGGATTCAAC

930
GACTACAAGACTGCTGTTGGAATCTTTGAC

950 970
ACTAACTACCAAAACGGTAGAGAAAATGCT

990
CCAATTGACTTTGAACAATTGATCACCCAC

1010 1030
AGATACAAGTTCAAGGACGCTATTGAAGCC

1050
TACGACTTGGTCAGAGCCGGTAAGGGTGCT

1070 1090
GTCAAGTGTCTCATTGACGGCCCTGAGTAA

1110
GTCAACCGCTTGGCTGGCCCAAAGTGAACC

1130 1150
AGAAACGAAAATGATTATCAAATAGCTTTA

1170
TAGACCTTTATCGAAATTTATGTAAACTAA

1190 1210
TAGAAAAGACAGTGTAGAAGTTATATGGTT

1230
GCATCACGTGAGTTTCTTGAATTCTTGAAA

1250 1270
GTGAAGTCTTGGTCGGAACAAACAAACAAA

1290
AAAATATTTTCAGCAAGAGTTGATTTCTTT

1310 1330
TCTGGAGATTTTGGTAATTGACAGAGAACC

1350
CCTTTCTGCTATTGCCATCTAAACATCCTT

1370 1390
GAATAGAACTTTACTGGATGGCCGCCTAGT

1410
GTTGAGTATATATTATCAACCAAAATCCTG

1430 1450
TATATAGTCTCTGAAAAATTTGACTATCCT

1470
AACTTAACAAAAGAGCACCATAATGCAAGC

1490 1510
TCATAGTTCTTAGAGACACCAACTATACTT

1530
AGCCAAACAAAATGTCCTTGGCCTCTAAAG

1550 1570
AAGCATTCAGCAAGCTTCCCCAGAAGTTGC

1590
ACAACTTCTTCATCAAGTTTACCCCCAGAC

1610 1630
CGTTTGCCGAATATTCGGAAAAGCCTTCGA

CTATAGTGGATCC

7. DNA-Sequenz nach einem der Ansprüche 1 bis 6, dadurch gekennzeichnet, daß sie durch rekombinante DNA-Technologie aus natürlicher DNA und/oder cDNA und/oder chemisch synthetisierter DNA erhalten wird.

8. Kombination von DNA-Sequenzen, dadurch gekennzeichnet, daß die Kombinantion eine erste DNA-Sequenz gemäß einem der Ansprüche 1 bis 7 und eine oder mehrere DNA-Sequenzen umfaßt, die die Expression eines von der DNA-Sequenz kodierten Strukturgenes in einem Wirtsorganismus regulieren kann.

9. Kombination von DNA-Sequenzen nach Anspruch 8, dadurch gekennzeichnet, daß die Kombination Modifikationen der DNA-Sequenzen umfaßt, die ihre Fähigkeit zur Expression eines funktionellen Enzymes mit Xylosereduktase- oder Xylitoldehydrogenase-Aktivität aufrecht erhält.

10. Kombination von DNA-Sequenzen nach Anspruch 8 oder 9, dadurch gekennzeichnet, daß das Strukturgen DNA-Sequenzen enthält, die von dem für Xylosereduktase oder Xylitoldehydrogenase kodierenden Strukturgen abgeleitet sind, die das Proteinprodukt modifizieren, während seine Funktionen auf eine solche Weise beibehalten werden, daß das Proteinprodukt als ein Genprodukt mit enzymatischer Aktivität exprimiert wird.

11. Kombination von DNA-Sequenzen nach einem der Ansprüche 8 bis 10, dadurch gekennzeichnet, daß die DNA-Sequenzen, die Expression des Strukturgenes in einem Wirtsorganismus regulieren können, von dem Wirtsorganismus abgeleitet sind.

12. Kombination nach Anspruch 11, dadurch gekennzeichnet, daß die DNA-Sequenzen, die die Expression regulieren können, induzierbare Promotoren sind.

13. Kombination nach Anspruch 12, dadurch gekennzeichnet, daß die DNA-Sequenzen, die die Expression regulieren können, aus den folgenden Promotoren ausgewählt sind:
ADH1, ADH2, PDC, GAL1/10.

14. Kombination nach einem der Ansprüche 11 bis 13, dadurch gekennzeichnet, daß die DNA-Sequenz, die die Expression des Strukturgenes regulieren kann, ein starker Promotor ist, was zur Überexpression des von dem Strukturgen kodierten Proteines führt.

15. Vektor, dadurch gekennzeichnet, daß der Vektor eine DNA-Sequenz nach einem der Ansprüche 1 bis 7 oder eine Kombination von DNA-Sequenzen nach einem der Ansprüche 8 bis 14 umfaßt.

16. Vektor nach Anspruch 15, dadurch gekennzeichnet, daß der Vektor aus der Gruppe ausgewählt ist, die die Plasmide pR1, pR2, pD1, pD2, pRD1, pXRa, pXRb, pXDH, pXR, pXDH-HIS3, pXR-LEU2 umfaßt.

17. Mikroorganismus, dadurch gekennzeichnet, daß der Mikroorganismus eine Xylosereduktase oder Xylosereduktase und Xylitolhydrogenase mittels rekombinanter DNA-Technologie exprimieren kann als Ergebnis dessen, daß er DNA-Sequenzen erhalten hat, die DNA-Sequenzen nach einem der Ansprüche 1 bis 7 oder eine Kombination von DNA-Sequenzen nach einem der Ansprüche 8 bis 14 umfassen, die für die Xylosereduktase oder die Xylosereduktase und Xylitolhydrogenase kodieren.

18. Mikroorganismus nach Anspruch 17, dadurch gekennzeichnet, daß der Mikroorganismus aus einer Gruppe ausgewählt ist, die aus Hefe der Gattungen Saccharomyces, Schizosaccharomyces, Schwanniomyces, Kluyveromyces, Pichia, Hansenula, Candida, Debaryomyces, Metschnikowia, Pachysolen oder Paecilomyces oder Bakterien der Gattung Zymomonas besteht.

19. Mikroorganismus nach Anspruch 18, dadurch gekennzeichnet, daß der Mikroorganismus Saccharomyces cerevisiae ist.

20. Mikroorganismus nach Anspruch 18, dadurch gekennzeichnet, daß der Mikroorganismus Schizosaccharomyces pombe ist.

21. Mikroorganismus nach einem der Ansprüche 17 bis 20, dadurch gekennzeichnet, daß die DNA-Sequenz oder eine Kombination von DNA-Sequenzen in das Genom des Mikroorganismus integriert ist.

22. Mikroorganismus nach einem der Ansprüche 17 bis 21, dadurch gekennzeichnet, daß der Mikroorganismus für die Erzeugung von Biomasse, in der Nahrungsmittelindustrie oder in Fermentationsverfahren nützlich ist.

23. Mikroorganismus nach Anspruch 22, dadurch gekennzeichnet, daß der Mikroorganismus für die Fermentation von Xylose in Ethanol nützlich ist.

24. Verfahren zum Erzeugen von Xylosereduktase oder Xylosereduktase und Xylitoldehydrogenase durch Kultivieren eines Mikroorganismus nach einem der Ansprüche 17 bis 21 unter geeigneten Bedingungen und Gewinnen des Enzymes (der Enzyme) in an sich bekannter Weise.

25. Verfahren nach Anspruch 24, dadurch gekennzeichnet, daß der Mikroorganismus für eine effiziente Fermentation von Xylulose ausgewählt wird.

26. Verfahren nach Anspruch 24 oder 25, dadurch gekennzeichnet, daß der Mikroorganismus die DNA-Sequenzen oder die Kombination von DNA-Sequenzen durch Transformation unter Verwendung eines Vektors erhalten hat, wobei der Vektor bevorzugt ein DNA-Fragment oder ein Plasmid ist.

27. Verfahren nach Anspruch 26, dadurch gekennzeichnet, daß der Vektor DNA enthält, die der DNA des Mikroorganismus homolog ist, was zur Integration in das Genom des Mikroorganismus führt.

28. Ethanol-Herstellungsverfahren, dadurch gekennzeichnet, daß ein Mikroorganismus gemäß einem der Ansprüche 17 bis 23 verwendet wird.

29. Verfahren nach Anspruch 28, dadurch gekennzeichnet, daß das Fermentationsverfahren an die Erzeugung von alkoholischen Getränken oder Einzelzellprotein angepaßt ist, die aus Substraten erzeugt werden, die freie Xylose enthalten, die bevorzugt durch Xylanase- und/oder Xylosidase-Aktivität freigesetzt wird.

30. Verfahren zum Erzeugen von Biomassen, dadurch gekennzeichnet, daß ein Wirtsorganismus gemäß einem der Ansprüche 17 bis 23 verwendet wird.

## Patentansprüche (Patentansprüche für folgende(n) Vertragsstaat(en): ES)

1. Verfahren zum Herstellen einer DNA-Sequenz, die ein für eine Xylosereduktase mit der folgenden Aminosäuresequenz kodierendes Strukturgen umfaßt: 
10
M P S I K L N G G Y
20
D M P A V G F G C W
30
K V D V D T C S E Q
40
I Y R A I K T G Y R
50
L F D G A E D Y A N
60
E K L V G A G V K K
70
A I D E G I V K R E
80
D L F L T S K L W N
90
N Y H H P D N V E K
100
A L N R T L S D L Q
110
V D Y V D L F L I H
120
F P V T F K F V P L
130
E E K Y P P G F Y C
140
G K G D N F D Y E D
 
150
V P I L E T W K A L
160
E K L V K A G K I R
170
S I G V S N F P G A
180
L L L D L L R G A T
190
I K P S V L Q V E H
200
H P Y L Q Q P R L I
210
E F A Q S R G I A V
220
T A Y S S F G P Q S
230
F V E L N Q G R A L
240
N T S P L F E N E T
250
I K A I A A K H G K
260
S P A Q V L L R W S
270
S Q R G I A I I P K
280
S N T V P R L L E N
 
290
K D V N S F D L D E
300
Q D F A D I A K L D
310
I N L R F N D P W D
W D K I P I F V *
 wobei die DNA-Sequenz das Polypeptid in einem Mikroorganismus exprimieren kann, wobei die DNA-Sequenz mittels rekombinanter DNA-Techologie aus natürlicher DNA und/oder cDNA und/oder chemisch synthetisierter DNA hergestellt wird.

2. Verfahren nach Anspruch 1, wobei die DNA-Sequenz weiter ein für Xylitoldehydrogenase mit der folgenden Aminosäuresequenz kodierendes Strukturgen umfaßt:

10
M T A N P S L V L N
20
K I D D I S F E T Y
30
D A P E I S E P T D
40
V L V Q V K K T G I
50
C G S D I H F Y A H
60
G R I G N F V L T K
70
P M V L G H E S A G
80
T V V Q V G K G V T
90
S L K V G D N V A I
100
E P G I P S R F S D
110
E Y K S G H Y N L C
120
P H M A F A A T P N
130
S K E G E P N P P G

140
T L C K Y F K S P E

150
D F L V K L P D H V

160
S L E L G A L V E P

170
L S V G V H A S K L

180
G S V A F G D Y V A

190
V F G A G P V G L L

200
A A A V A K T F G A

210
K G V I V V D I F D

220
N K L K M A K D I G

230
A A T H T F N S K T

240
G G S E E L I K A F

250
G G N V P N V V L E

260
C T G A E P C I K L

270
G V D A I A P G G R
280
F V Q V G N A A G P
290
V S F P I T V F A M
300
K E L T L F G S F R
310
Y G F N D Y K T A V
320
G I F D T N Y Q N G
330
R E N A P I D F E Q
340
L I T H R Y K F K D
350
A I E A Y D L V R A
360
G K G A V K C L I D
G P E *

3. Verfahren nach einem der Ansprüche 1 oder 2, dadurch gekennzeichnet, daß die DNA-Sequenz von einer Hefe abgeleitet ist, bevorzugt von einer Hefe, die aus der Gruppe ausgewählt ist, die aus den Gattungen Schwanniomyces, Saccharomyces, Kluyveromyces, Pichia, Hansenula, Candida, Debaryomyces, Metschnikowia und Pachysolen besteht.

4. Verfahren nach Anspruch 3, dadurch gekennzeichnet, daß die Hefe Pichia stipitis ist, bevorzugt Pichia stipitis CBS 5773 (DSM 5855).

5. Verfahren nach Anspruch 1, wobei die DNA-Sequenz die folgende Nukleotidsequenz umfaßt:

-350
GGATCCACAGACACTAATTGGTTCTA
-310
CATTATTCGTGTTCAGACACAAACCCCAGC
-290
GTTGGCGGTTTCTGTCTGCGTTCCTCCAGC
-250
ACCTTCTTGCTCAACCCCAGAAGGTGCACA
-230
CTGCAGACACACATACATACGAGAACCTGG
-190
AACAAATATCGGTGTCGGTGACCGAAATGT
-170
GCAAACCCAGACACGACTAATAAACCTGGC
-130
AGCTCCAATACCGCCGACAACAGGTGAGGT
-110
GACCGATGGGGTGCCAATTAATGTCTGAAA
-70
ATTGGGGTATATAAATATGGCGATTCTCCG
-50
GAGAATTTTTCAGTTTCTTTTCATTTCTC
-10
CAGTATTCTTTTCTATACAACTATACTACA
10 30
ATGCCTTCTATTAAGTTGAACTCTGGTTAC
50
GACATGCCAGCCGTCGGTTTCGGCTGTTGG

70 90
AAAGTCGACGTCGACACCTGTTCTGAACAG

110
ATCTACCGTGCTATCAAGACCGGTTACAGA

130 150
TTGTTCGACGGTGCCGAAGATTACGCCAAC

170
GAAAAGTTAGTTGGTGCCGGTGTCAAGAAG

190 210
GCCATTGACGAAGGTATCGTCAAGCGTGAA

230
GACTTGTTCCTTACCTCCAAGTTGTGGAAC

250 270
AACTACCACCACCCAGACAACGTCGAAAAG

290
GCCTTGAACAGAACCCTTTCTGACTTGCAA

310 330
GTTGACTACGTTGACTTGTTCTTGATCCAC

350
TTCCCAGTCACCTTCAAGTTCGTTCCATTA

370 390
GAAGAAAAGTACCCACCAGGATTCTACTGT

410
GGTAAGGGTGACAACTTCGACTACGAAGAT

430 450
GTTCCAATTTTAGAGACCTGGAAGGCTCTT

470
GAAAAGTTGGTCAAGGCCGGTAAGATCAGA

490 510
TCTATCGGTGTTTCTAACTTCCCAGGTGCT

530
TTGCTCTTGGACTTGTTGAGAGGTGCTACC

550 570
ATCAAGCCATCTGTCTTGCAAGTTGAACAC

590
CACCCATACTTGCAACAACCAAGATTGATC

610 630
GAATTCGCTCAATCCCGTGGTATTGCTGTC

650
ACCGCTTACTCTTCGTTCGGTCCTCAATCT

670 690
TTCGTTGAATTGAACCAAGGTAGAGCTTTG

710
AACACTTCTCCATTGTTCGAGAACGAAACT

730 750
ATCAAGGCTATCGCTGCTAAGCACGGTAAG

770
TCTCCAGCTCAAGTCTTGTTGAGATGGTCT

790 810
TCCCAAAGAGGCATTGCCATCATTCCAAAG

830
TCCAACACTGTCCCAAGATTGTTGGAAAAC

850 870
AAGGACGTCAACAGCTTCGACTTGGACGAA

890
CAAGATTTCGCTGACATTGCCAAGTTGGAC

910 930
ATCAACTTGAGATTCAACGACCCATGGGAC

950
TGGGACAAGATTCCTATCTTCGTCTAAGAA

970 990
GGTTGCTTTATAGAGAGGAAATAAAACCTA

1010
ATATACATTGATTGTACATTTAAAATTGAA

1030 1050
TATTGTAGCTAGCAGATTCGGAAATTTAAA

1070
ATGGAAGGTGATTCTATCCGTACGAATGA

1090 1110
TCTCTATGTACATACACGTTGAAGATAGCA

1130
GTACAGTAGACATCAAGTCTACAGATCATT

1150 1170
AAACATATCTTAAATTGTAGAAAACTATAA

1190
ACTTTTCAATTCAAACCATGTCTGCCAAGG

1210 1230
AATCAAATGAGATTTTTTCGCAGCCAAAC

1250
TTGAATCCAAAAATAAAAACGTCATTGTC

1270 1290
TGAAACAACTCTATCTTATCTTTCACCTCA

1310
TCAATTCATTGCATATCATAAAAGCCTCCG

1330                                    1350
ATAGCATACAAAACTACTTCTGCATCATAT

                    1370
CTAAATCATAGTGCCATATTCAGTAACAAT

1390                                    1410
ACCGGTAAGAAACTTCTATTTTTTTAGTCT

                    1430
GCCTTAACGAGATGCAGATCGATGCAACGT

1450                                    1470
AAGATCAAACCCCTCCAGTTGTACAGTCAG

                    1490
TCATATAGTGAACACCGTACAATATGGTAT

1510                                    1530
CTACGTTCAAATAGACTCCAATACAGCTGG

                    1550
TCTGCCCAAGTTGAGCAACTTTAATTTAGA

1570                                    1590
GACAAAGTCGTCTCTGTTGATGTAGGCACC

                    1610
ACACATTCTTCTCTTGCCCGTGAACTCTGT

1630                                    1650
TCTGGAGTGGAAACATCTCCAGTTGTCAAA

                    1670
TATCAAACACTGACCAGGCTTCAACTGGTA

1690
GAAGATTTCGTTTTCGGGATC

6. Verfahren nach Anspruch 2, wobei die DNA-Sequenz die folgenden Nukleotidsequenz umfaßt:

-310                                    -290
TCTAGACCACCCTAAGTCGTCCCTATGTCG

                    -270
TATGTTTGCCTCTACTACAAAGTTACTAGC

-250                                    -230
AAATATCCGCAGCAACAACAGCTGCCCTCT

                    -210
TCCAGCTTCTTAGTGTGTTGGCCGAAAAGG

-190                                    -170
CGCTTTCGGGCTCCAGCTTCTGTCCTCTGC

                    -150
GGCTGCTGCACATAACGCGGGACAATGAC

-130                                    -110
TTCTCCAGCTTTTATTATAAAAGGAGCCAT

                    -90
CTCCTCCAGGTGAAAAATTACGATCAACTT

-70                                     -50
TTACTCTTTTCCATTGTCTCTTGTGTATAC

                    -30
TCACTTTAGTTTGTTTCAATCACCCCTAAT

-10                                     10
ACTCTTCACACAATTAAAATGACTGCTAAC

                    30
CCTTCCTTGGTGTTGAACAAGATCGACGAC

50                                      70
ATTTCGTTCGAAACTTACGATGCCCCAGAA

90
ATCTCTGAACCTACCGATGTCCTCGTCCAG

110 130
GTCAAGAAAACCGGTATCTGTGGTTCCGAC

150
ATCCACTTCTACGCCCATGGTAGAATCGGT

170 190
AACTTCGTTTTGACCAAGCCAATGGTCTTG

210
GGTCACGAATCCGCCGGTACTGTTGTCCAG

230 250
GTTGGTAAGGGTGTCACCTCTCTTAAGGTT

270
GGTGACAACGTCGCTATCGAACCAGGTATT

290 310
CCATCCAGATTCTCCGACGAATACAAGAGC

330
GGTCACTACAACTTGTGTCCTCACATGGCC

350 370
TTCGCCGCTACTCCTAACTCCAAGGAAGGC

390
GAACCAAACCCACCAGGTACCTTATGTAAG

410 430
TACTTCAAGTCGCCAGAAGACTTCTTGGTC

450
AAGTTGCCAGACCACGTCAGCTTGGAACTC

470 490
GGTGCTCTTGTTGAGCCATTGTCTGTTGGT

510
GTCCACGCCTCCAAGTTGGGTTCCGTTGCT

530 550
TTCGGCGACTACGTTGCCGTCTTTGGTGCT

570
GGTCCTGTTGGTCTTTTGGCTGCTGCTGTC

590 610
GCCAAGACCTTCGGTGCTAAGGGTGTCATC

630
GTCGTTGACATTTTCGACAACAAGTTGAAG

650 670
ATGGCCAAGGACATTGGTGCTGCTACTCAC

690
ACCTTCAACTCCAAGACCGGTGGTTCTGAA

710 730
GAATTGATCAAGGCTTTCGGTGGTAACGTG

750
CCAAACGTCGTTTTGGAATGTACTGGTGCT

770 790
GAACCTTGTATCAAGTTGGGTGTTGACGCC

810
ATTGCCCCAGGTGGTCGTTTCGTTCAAGTT

830 850
GGTAACGCTGCTGGTCCAGTCAGCTTCCCA

870
ATCACCGTTTTCGCCATGAAGGAATTGACT

890 910
TTGTTCGGTTCTTTCAGATACGGATTCAAC

930
GACTACAAGACTGCTGTTGGAATCTTTGAC

950 970
ACTAACTACCAAAACGGTAGAGAAAATGCT

990
CCAATTGACTTTGAACAATTGATCACCCAC

1010 1030
AGATACAAGTTCAAGGACGCTATTGAAGCC

1050
TACGACTTGGTCAGAGCCGGTAAGGGTGCT

1070 1090
GTCAAGTGTCTCATTGACGGCCCTGAGTAA

1110
GTCAACCGCTTGGCTGGCCCAAAGTGAACC

1130 1150
AGAAACGAAAATGATTATCAAATAGCTTTA

1170
TAGACCTTTATCGAAATTTATGTAAACTAA

1190 1210
TAGAAAAGACAGTGTAGAAGTTATATGGTT

1230
GCATCACGTGAGTTTCTTGAATTCTTGAAA

1250                                    1270
GTGAAGTCTTGGTCGGAACAAACAAACAAA

                    1290
AAAATATTTTCAGCAAGAGTTGATTTCTTT

1310                                    1330
TCTGGAGATTTTGGTAATTGACAGAGAACC

                    1350
CCTTTCTGCTATTGCCATCTAAACATCCTT

1370                                    1390
GAATAGAACTTTACTGGATGGCCGCCTAGT

                    1410
GTTGAGTATATATTATCAACCAAAATCCTG

1430                                    1450
TATATAGTCTCTGAAAAATTTGACTATCCT

                    1470
AACTTAACAAAAGAGCACCATAATGCAAGC

1490                                    1510
TCATAGTTCTTAGAGACACCAACTATACTT

                    1530
AGCCAAACAAAATGTCCTTGGCCTCTAAAG

1550                                    1570
AAGCATTCAGCAAGCTTCCCCAGAAGTTGC

                    1590
ACAACTTCTTCATCAAGTTTACCCCCAGAC

1610                                    1630
CGTTTGCCGAATATTCGGAAAAGCCTTCGA

CTATAGTGGATCC

7. Verfahren zum Herstellen einer Kombination von DNA-Sequenzen, wobei das Verfahren das Vereinen einer ersten DNA-Sequenz, erhältlich gemäß einem der Ansprüche 1 bis 6, und einer oder mehrerer weiterer DNA-Sequenzen, die die Expression eines von der DNA-Sequenz kodierten Strukturgenes in einem Wirtsorganismus regulieren können, in an sich bekannter Weise umfaßt.

8. Verfahren nach Anspruch 7, wobei die Kombination von Sequenzen Modifikationen der DNA-Sequenzen umfaßt, die deren Fähigkeit zur Expression eines funktionellen Enzymes mit Xylosereduktase- oder Xylitoldehydrogenase-Aktivität aufrecht erhält.

9. Verfahren nach einem der Ansprüche 7 oder 8, wobei das Strukturgen DNA-Sequenzen enthält, die von dem für Xylosereduktase oder Xylitoldehydrogenase kodierenden Strukturgen abgeleitet sind, die das Proteinprodukt modifizieren, während sie seine Funktionen auf eine solche Weise aufrecht erhalten, daß das Proteinprodukt als ein Genprodukt mit enzymatischer Aktivität exprimiert wird.

10. Verfahren nach einem der Ansprüche 7 bis 9, wobei die DNA-Sequenzen, die die Expression des Strukturgenes in einem Wirtsorganismus regulieren können, von dem Wirtsorganismus abgeleitet sind.

11. Verfahren nach Anspruch 10, wobei die DNA-Sequenzen, die die Expression regulieren können, induzierbare Promotoren sind.

12. Verfahren nach Anspruch 11, dadurch gekennzeichnet, daß die DNA-Sequenzen, die die Expression regulieren können, aus den folgenden Promotoren ausgewählt sind:
ADH1, ADH2, PDC, GAL1/10.

13. Verfahren nach einem der Ansprüche 10 bis 12, wobei die DNA-Sequenzen, die die Expression des Strukturgenes regulieren können, starke Promotor sind, was zur Überexpression des von dem Strukturgen kodierten Proteines führt.

14. Verfahren zum Herstellen eines Vektors, wobei das Verfahren das Insertieren einer DNA-Sequenz, die gemäß einem der Ansprüche 1 bis 6 erhältlich ist, oder einer Kombination von DNA-Sequenzen, die gemäß einem der Ansprüche 7 bis 13 erhältlich ist, in ein Wirtsplasmid umfaßt.

15. Verfahren nach Anspruch 14, dadurch gekennzeichnet, daß es einen Vektor erzeugt, der aus der Gruppe ausgewählt ist, die die Plasmide pR1, pR2, pD1, pD2, pRD1, pXRa, pXRb, pXDH, pXR, pXDH-HIS3, pXR-LEU2 umfaßt.

16. Verfahren zum Herstellen eines Mikroorganismus, der Xylosereduktase oder Xylosereduktase und Xylitolhydrogenase exprimieren kann, wobei DNA-Sequenzen, die die gemäß einem der Ansprüche 1 bis 6 erhältlichen DNA-Sequenzen oder eine Kombination von DNA-Sequenzen, die gemäß einem der Ansprüche 7 bis 13 erhältlich ist, und die für die Xylosereduktase oder die Xylosereduktase und Xylitolhydrogenase kodieren, umfassen, in einen Wirtsmikroorganismus eingeführt werden.

17. Verfahren nach Anspruch 16, dadurch gekennzeichnet, daß der Wirtsmikroorganismus aus einer Gruppe ausgewählt ist, die aus Hefen der Gattungen Saccharomyces, Schizosaccharomyces, Schwanniomyces, Kluyveromyces, Pichia, Hansenula, Candida, Debaryomyces, Metschnikowia, Pachysolen oder Paecilomyces oder Bakterien der Gattung Zymomonas besteht.

18. Verfahren nach Anspruch 17, dadurch gekennzeichnet, daß der Mikroorganismus Saccharomyces cerevisiae ist.

19. Verfahren nach Anspruch 17, dadurch gekennzeichnet, daß der Mikroorganismus Schizosaccharomyces pombe ist.

20. Verfahren nach einem der Ansprüche 16 bis 19, dadurch gekennzeichnet, daß die DNA-Sequenz oder eine Kombination von DNA-Sequenzen in das Genom des Mikroorganismus integriert wird.

21. Verfahren nach einem der Ansprüche 17 bis 21, dadurch gekennzeichnet, daß der Mikroorganismus bei der Erzeugung von Biomasse, in der Nahrungsmittelindustrie oder bei Fermentationsverfahren nützlich ist.

22. Verfahren nach Anspruch 21, dadurch gekennzeichnet, daß der Mikroorganismus für die Fermentation von Xylose in Ethanol nützlich ist.

23. Verfahren zum Erzeugen von Xylosereduktase oder Xylosereduktase und Xylitoldehydrogenase durch Kultivieren eines Mikroorganismus, der gemäß einem der Ansprüche 16 bis 20 erhältlich ist, unter geeigneten Bedingungen und Gewinnen des Enzymes (der Enzyme) in an sich bekannter Weise.

24. Verfahren nach Anspruch 23, dadurch gekennzeichnet, daß der Mikroorganismus für effiziente Fermentation von Xylulose ausgewählt wird.

25. Verfahren nach Anspruch 23 oder 24, dadurch gekennzeichnet, daß der Mikroorganismus die DNA-Sequenzen oder die Kombination von DNA-Sequenzen durch Transformation unter Verwendung eines Vektors erhalten hat, wobei der Vektor bevorzugt ein DNA-Fragment oder ein Plasmid ist.

26. Verfahren nach Anspruch 25, dadurch gekennzeichnet, daß der Vektor DNA enthält, die der DNA des Mikroorganismus homolog ist, was zur Integration in das Genom des Mikroorganismus führt.

27. Ethanol-Herstellungsverfahren, dadurch gekennzeichnet, daß ein nach einem der Ansprüche 16 bis 22 erhältlicher Mikroorganismus verwendet wird.

28. Verfahren nach Anspruch 27, dadurch gekennzeichnet, daß das Fermentationsverfahren an die Erzeugung von alkoholischen Getränken oder Einzelzellprotein angepaßt ist, die aus Substraten erzeugt werden, die freie Xylose enthalten, die bevorzugt durch Xylanase und/oder Xylosidase-Aktivität freigesetzt wird.

29. Verfahren zum Erzeugen von Biomasse, dadurch gekennzeichnet, daß ein Wirtsorganismus nach einem der Ansprüche 16 bis 22 verwendet wird.

## Revendications (Revendications pour l'(les) Etat(s) contractant(s) suivant(s): AT, BE, CH, LI, DE, DK, FR, GB, GR, IT, LU, NL, SE)

1. Séquence d'ADN, caractérisée en ce que ladite séquence d'ADN comprend un gène de structure codant pour une réductase du xylose ayant la séquence suivante d'acides aminés : 
10
M P S I K L N S G Y
20
D M P A V G F G C W
30
K V D V D T C S E Q
40
I Y R A I K T G Y R
50
L F D G A E D Y A N
60
E K L V G A G V K K
70
A I D E G I V K R E
80
D L F L T S K L W N
90
N Y H H P D N V E K
100
A L N R T L S D L Q
110
V D Y V D L F L I H
120
F P V T F K F V P L
130
E E K Y P P G F Y C
140
G K G D N F D Y E D
 
150
V P I L E T W K A L
160
E K L V K A G K I R
170
S I G V S N F P G A
180
L L L D L L R G A T
190
I K P S V L Q V E H
200
H P Y L Q Q P R L I
210
E F A Q S R G I A V
220
T A Y S S F G P Q S
230
F V E L N Q G R A L
240
N T S P L F E N E T
250
I K A I A A K H G K
260
S P A Q V L L R W S
270
S Q R G I A I I P K
280
S N T V P R L L E N
 
290
K D V N S F D L D E
300
Q D F A D I A K L D
310
I N L R F N D P W D
W D K I P I F V *
 dans laquelle ladite séquence d'ADN est capable d'exprimer ledit polypeptide dans un micro-organisme.

2. Séquence d'ADN selon la revendication 1, caractérisée en ce que ladite séquence d'ADN comprend en outre un gène de structure codant pour la déshydrogénase du xylitol ayant la séquence suivante d'acides aminés :

10
M T A N P S L V L N
20
K I D D I S F E T Y
30
D A P E I S E P T D
40
V L V Q V K K T G I
50
C G S D I H F Y A H
60
G R I G N F V L T K
70
P M V L G H E S A G
80
T V V Q V G K G V T
90
S L K V G D N V A I
100
E P G I P S R F S D
110
E Y K S G H Y N L C
120
P H M A F A A T P N
130
S K E G E P N P P G

140
T L C K Y F K S P E
150
D F L V K L P D H V
160
S L E L G A L V E P
170
L S V G V H A S K L
180
G S V A F G D Y V A
190
V F G A G P V G L L
200
A A A V A K T F G A
210
K G V I V V D I F D
220
N K L K M A K D I G
230
A A T H T F N S K T
240
G G S E E L I K A F
250
G G N V P N V V L E
260
C T G A E P C I K L

270
G V D A I A P G G R
280
F V Q V G N A A G P
290
V S F P I T V F A M
300
K E L T L F G S F R
310
Y G F N D Y K T A V
320
G I F D T N Y Q N G
330
R E N A P I D F E Q
340
L I T H R Y K F K D
350
A I E A Y D L V R A
360
G K G A V K C L I D
G P E *

3. Séquence d'ADN selon les revendications 1 ou 2, caractérisée- en ce que ladite séquence d'ADN est dérivée d'une levure, de préférence d'une levure choisie parmi un groupe constitué des genres Schwanniomyces, Saccharomyces, Kluyveromyces, Pichia, Hansenula, Candida, Debaryomyces, Metschnikowia, et Pachysolen.

4. Séquence d'ADN selon la revendication 3, caractérisée en ce que la levure est Pichia stipitis, de préférence Pichia stipitis CBS 5773 (DSM 5855).

5. Séquence d'ADN selon la revendication 1, comprenant la séquence suivante de nucléotides :

-350
GGATCCACAGACACTAATTGGTTCTA

-310
CATTATTCGTGTTCAGACACAAACCCCAGC

-290
GTTGGCGGTTTCTGTCTGCGTTCCTCCAGC

-250
ACCTTCTTGCTCAACCCCAGAAGGTGCACA

-230
CTGCAGACACACATACATACGAGAACCTGG

-190
AACAAATATCGGTGTCGGTGACCGAAATGT

-170
GCAAACCCAGACACGACTAATAAACCTGGC

-130
AGCTCCAATACCGCCGACAACAGGTGAGGT

-110
GACCGATGGGGTGCCAATTAATGTCTGAAA

-70
ATTGGGGTATATAAATATGGCGATTCTCCG

-50
GAGAATTTTTCAGTTTTCTTTTCATTTCTC

-10
CAGTATTCTTTTCTATACAACTATACTACA

10 30
ATGCCTTCTATTAAGTTGAACTCTGGTTAC

50
GACATGCCAGCCGTCGGTTTCGGCTGTTGG

70 90
AAAGTCGACGTCGACACCTGTTCTGAACAG

110
ATCTACCGTGCTATCAAGACCGGTTACAGA

130 150
TTGTTCGACGGTGCCGAAGATTACGCCAAC

170
GAAAAGTTAGTTGGTGCCGGTGTCAAGAAG

190 210
GCCATTGACGAAGGTATCGTCAAGCGTGAA

230
GACTTGTTCCTTACCTCCAAGTTGTGGAAC

250 270
AACTACCACCACCCAGACAACGTCGAAAAG

290
GCCTTGAACAGAACCCTTTCTGACTTGCAA

310 330
GTTGACTACGTTGACTTGTTCTTGATCCAC

350
TTCCCAGTCACCTTCAAGTTCGTTCCATTA

370 390
GAAGAAAAGTACCCACCAGGATTCTACTGT

410
GGTAAGGGTGACAACTTCGACTACGAAGAT

430 450
GTTCCAATTTTAGAGACCTGGAAGGCTCTT

470
GAAAAGTTGGTCAAGGCCGGTAAGATCAGA

490 510
TCTATCGGTGTTTCTAACTTCCCAGGTGCT

530
TTGCTCTTGGACTTGTTGAGAGGTGCTACC

550 570
ATCAAGCCATCTGTCTTGCAAGTTGAACAC

590
CACCCATACTTGCAACAACCAAGATTGATC

610 630
GAATTCGCTCAATCCCGTGGTATTGCTGTC

650
ACCGCTTACTCTTCGTTCGGTCCTCAATCT

670 690
TTCGTTGAATTGAACCAAGGTAGAGCTTTG

710
AACACTTCTCCATTGTTCGAGAACGAAACT

730 750
ATCAAGGCTATCGCTGCTAAGCACGGTAAG

770
TCTCCAGCTCAAGTCTTGTTGAGATGGTCT

790 810
TCCCAAAGAGGCATTGCCATCATTCCAAAG

830
TCCAACACTGTCCCAAGATTGTTGGAAAAC

850 870
AAGGACGTCAACAGCTTCGACTTGGACGAA

890
CAAGATTTCGCTGACATTGCCAAGTTGGAC

910 930
ATCAACTTGAGATTCAACGACCCATGGGAC

950
TGGGACAAGATTCCTATCTTCGTCTAAGAA

970 990
GGTTGCTTTATAGAGAGGAAATAAAACCTA

1010
ATATACATTGATTGTACATTTAAAATTGAA

1030 1050
TATTGTAGCTAGCAGATTCGGAAATTTAAA

1070
ATGGAAGGTGATTCTATCCGTACGAATGA

1090 1110
TCTCTATGTACATACACGTTGAAGATAGCA

1130
GTACAGTAGACATCAAGTCTACAGATCATT

1150 1170
AAACATATCTTAAATTGTAGAAAACTATAA

1190
ACTTTTCAATTCAAACCATGTCTGCCAAGG

1210 1230
AATCAAATGAGATTTTTTTCGCAGCCAAAC

1250
TTGAATCCAAAAATAAAAAACGTCATTGTC

1270 1290
TGAAACAACTCTATCTTATCTTTCACCTCA

1310
TCAATTCATTGCATATCATAAAAGCCTCCG

1330 1350
ATAGCATACAAAACTACTTCTGCATCATAT

1370
CTAAATCATAGTGCCATATTCAGTAACAAT

1390 1410
ACCGGTAAGAAACTTCTATTTTTTTAGTCT

1430
GCCTTAACGAGATGCAGATCGATGCAACGT

1450 1470
AAGATCAAACCCCTCCAGTTGTACAGTCAG

1490
TCATATAGTGAACACCGTACAATATGGTAT

1510 1530
CTACGTTCAAATAGACTCCAATACAGCTGG

1550
TCTGCCCAAGTTGAGCAACTTTAATTTAGA

1570 1590
GACAAAGTCGTCTCTGTTGATGTAGGCACC

1610
ACACATTCTTCTCTTGCCCGTGAACTCTGT

1630 1650
TCTGGAGTGGAAACATCTCCAGTTGTCAAA

1670
TATCAAACACTGACCAGGCTTCAACTGGTA

1690
GAAGATTTCGTTTTCGGATC

6. Séquence d'ADN selon la revendication 2, comprenant la séquence suivante de nucléotides :

-310                                    -290
TCTAGACCACCCTAAGTCGTCCCTATGTCG

                        -270
TATGTTTGCCTCTACTACAAAGTTACTAGC

-250                                    -230
AAATATCCGCAGCAACAACAGCTGCCCTCT

                        -210
TCCAGCTTCTTAGTGTGTTGGCCGAAAAGG

-190                                    -170
CGCTTTCGGGCTCCAGCTTCTGTCCTCTGC

                        -150
GGCTGCTGCACATAACGCGGGACAATGAC

-130                                    -110
TTCTCCAGCTTTTATTATAAAGGAGCCAT

                        -90
CTCCTCCAGGTGAAAAATTACGATCAACTT

-70                                     -50
TTACTCTTTTCCATTGTCTCTTGTGTATAC

                        -30
TCACTTTAGTTTGTTTCAATCACCCCTAAT

-10                                     10
ACTCTTCACACAATTAAAATGACTGCTAAC

                        30
CCTTCCTTGGTGTTGAACAAGATCGACGAC

50                                      70
ATTTCGTTCGAAACTTACGATGCCCCAGAA

90
ATCTCTGAACCTACCGATGTCCTCGTCCAG

110 130
GTCAAGAAAACCGGTATCTGTGGTTCCGAC

150
ATCCACTTCTACGCCCATGGTAGAATCGGT

170 190
AACTTCGTTTTGACCAAGCCAATGGTCTTG

210
GGTCACGAATCCGCCGGTACTGTTGTCCAG

230 250
GTTGGTAAGGGTGTCACCTCTCTTAAGGTT

270
GGTGACAACGTCGCTATCGAACCAGGTATT

290 310
CCATCCAGATTCTCCGACGAATACAAGAGC

330
GGTCACTACAACTTGTGTCCTCACATGGCC

350 370
TTCGCCGCTACTCCTAACTCCAAGGAAGGC

390
GAACCAAACCCACCAGGTACCTTATGTAAG

410 430
TACTTCAAGTCGCCAGAAGACTTCTTGGTC

450
AAGTTGCCAGACCACGTCAGCTTGGAACTC

470 490
GGTGCTCTTGTTGAGCCATTGTCTGTTGGT

510
GTCCACGCCTCCAAGTTGGGTTCCGTTGCT

530 550
TTCGGCGACTACGTTGCCGTCTTTGGTGCT

570
GGTCCTGTTGGTCTTTTGGCTGCTGCTGTC

590 610
GCCAAGACCTTCGGTGCTAAGGGTGTCATC

630
GTCGTTGACATTTTCGACAACAAGTTGAAG

650 670
ATGGCCAAGGACATTGGTGCTGCTACTCAC

690
ACCTTCAACTCCAAGACCGGTGGTTCTGAA

710 730
GAATTGATCAAGGCTTTCGGTGGTAACGTG

750
CCAAACGTCGTTTTGGAATGTACTGGTGCT

770 790
GAACCTTGTATCAAGTTGGGTGTTGACGCC

810
ATTGCCCCAGGTGGTCGTTTCGTTCAAGTT

830 850
GGTAACGCTGCTGGTCCAGTCAGCTTCCCA

870
ATCACCGTTTTCGCCATGAAGGAATTGACT

890 910
TTGTTCGGTTCTTTCAGATACGGATTCAAC

930
GACTACAAGACTGCTGTTGGAATCTTTGAC

950 970
ACTAACTACCAAAACGGTAGAGAAAATGCT

990
CCAATTGACTTTGAACAATTGATCACCCAC

1010 1030
AGATACAAGTTCAAGGACGCTATTGAAGCC

1050
TACGACTTGGTCAGAGCCGGTAAGGGTGCT

1070 1090
GTCAAGTGTCTCATTGACGGCCCTGAGTAA

1110
GTCAACCGCTTGGCTGGCCCAAAGTGAACC

1130 1150
AGAAACGAAAATGATTATCAAATAGCTTTA

1170
TAGACCTTTATCGAAATTTATGTAAACTAA

1190 1210
TAGAAAAGACAGTGTAGAAGTTATATGGTT

1230
GCATCACGTGAGTTTCTTGAATTCTTGAAA

1250 1270
GTGAAGTCTTGGTCGGAACAAACAAACAAA

1290
AAAATATTTTCAGCAAGAGTTGATTTCTTT

1310 1330
TCTGGAGATTTTGGTAATTGACAGAGAACC

1350
CCTTTCTGCTATTGCCATCTAAACATCCTT

1370 1390
GAATAGAACTTTACTGGATGGCCGCCTAGT

1410
GTTGAGTATATATTATCAACCAAAATCCTG

1430 1450
TATATAGTCTCTGAAAAATTTGACTATCCT

1470
AACTTAACAAAAGAGCACCATAATGCAAGC

1490 1510
TCATAGTTCTTAGAGACACCAACTATACTT

1530
AGCCAAACAAAATGTCCTTGGCCTCTAAAG

1550 1570
AAGCATTCAGCAAGCTTCCCCAGAAGTTGC

1590
ACAACTTCTTCATCAAGTTTACCCCCAGAC

1610 1630
CGTTTGCCGAATATTCGGAAAAGCCTTCGA

CTATAGTGGATCC

7. Séquence d'ADN selon l'une quelconque des revendications 1 à 6, caractérisée en ce qu'elle est obtenue par la technologie de l'ADN recombinant, à partir d'ADN naturel et/ou d'ADNc et/ou d'ADN synthétisé chimiquement.

8. Combinaison de séquences d'ADN, caractérisée en ce que ladite combinaison comprend une première séquence d'ADN selon l'une quelconque des revendications 1 à 7 et une ou plusieurs autres séquences d'ADN capable de réguler l'expression d'un gène de structure encodé par ladite séquence d'ADN dans un micro-organisme hôte.

9. Combinaison de séquences d'ADN selon la revendication 8, caractérisée en ce que ladite combinaison comprend des modifications des séquences d'ADN conservant leur capacité à exprimer une enzyme fonctionnelle ayant une activité de réductase du xylose ou de déshydrogénase du xylitol.

10. Combinaison de séquences d'ADN selon la Revendication 8 ou 9, caractérisée en ce que ledit gène de structure contient des séquences d'ADN dérivées du gène de structure codant pour la réductase du xylose ou la déshydrogénase du xylitol qui modifie ledit produit de protéine tout en conservant ses fonctions d'une façon telle que ledit produit de protéine est exprimé comme un produit de gène ayant une activité enzymatique.

11. Combinaison de séquences d'ADN selon l'une quelconque des revendications 8 à 10, caractérisée en ce que lesdites séquences d'ADN capables de réguler l'expression dudit gène de structure dans un micro-organisme hôte, sont dérivées dudit micro-organisme hôte.

12. Combinaison selon la revendication 11, caractérisé en ce que lesdites séquences d'ADN capables de réguler l'expression sont des promoteurs susceptibles d'être induits.

13. Combinaison selon la revendication 12, caractérisée en ce que lesdites séquences d'ADN capables de réguler l'expression sont choisies parmi les promoteurs suivants :
ADH1, ADH2, PDC, GAL1/10.

14. Combinaison selon l'une quelconque des revendications 11 à 13, caractérisée en ce que ladite séquence d'ADN capable de réguler l'expression dudit gène de structure, est un promoteur fort, conduisant à une surexpression de la protéine encodée par ledit gène de structure.

15. Vecteur, caractérisé en ce que ledit vecteur comprend une séquence d'ADN selon l'une quelconque des revendications 1 à 7 ou une combinaison de séquences d'ADN selon l'une quelconque des revendications 8 à 14.

16. Vecteur selon la revendication 15, caractérisé en ce que ledit vecteur est choisi parmi le groupe comprenant les plasmides pR1, pR2, pD1, pD2, pRD1, pXRa, pXRb, pXDH, pXR, pXDH-HIS3, pXR-LEU2.

17. Micro-organisme, caractérisé en ce que ledit micro-organisme est capable d'exprimer une réductase du xylose ou une réductase du xylose et une deshydrogènase du xylitol après avoir reçu des séquences d'ADN comprenant les séquences d'ADN selon l'une quelconque des revendications 1 à 7 ou une combinaison de séquences d'ADN selon l'une quelconque des revendications 8 à 14, codant pour ladite réductase du xylose ou ladite réductase du xylose et ladite deshydrogènase du xylitol, par la technologie de l'ADN recombinant.

18. Micro-organisme selon la revendication 17, caractérisé en ce que ledit micro-organisme est choisi parmi un groupe constitué de levures des genres Saccharomyces, Schizosaccharomyces, Schwanniomyces, Kluyveromyces, Pichia, Hansenula, Candida, Debaryomyces, Metschnikowia, Pachysolen ou Paecilomyces ou de bactéries du genre Zymomonas.

19. Micro-organisme selon la revendication 18, caractérisé en ce que ledit micro-organisme est Saccharomyces cerevisiae.

20. Micro-organisme selon la revendication 18, caractérisé en ce que ledit micro-organisme est Schizosaccharomyces pombe.

21. Micro-organisme selon l'une quelconque des revendications 17 à 20, caractérisé en ce que ladite séquence d'ADN ou la combinaison de séquences d'ADN est intégrée dans le génome dudit micro-organisme.

22. Micro-organisme selon l'une quelconque des revendications 17 à 21, caractérisé en ce que ledit micro-organisme est utile dans la production de biomasse, dans l'industrie alimentaire et dans les procédés de fermentation.

23. Micro-organisme selon la revendication 22, caractérisé en ce que ledit micro-organisme est utile pour la fermentation du xylose dans l'éthanol.

24. Procédé de production de la réductase du xylose ou de la réductase du xylose et de la déshydrogénase du xylitol par culture d'un micro-organisme selon l'une quelconque des revendications 17 à 21 sous des conditions appropriées et récupération du(des)dits enzyme(s) d'une manière connue en soi.

25. Procédé selon la revendication 24, caractérisé en ce que ledit micro-organisme est choisi pour la fermentation efficace du xylulose.

26. Procédé selon la revendication 24 ou 25, caractérisé en ce que ledit micro-organisme a reçu lesdites séquences d'ADN ou ladite combinaison de séquences d'ADN par transformation utilisant un vecteur, ledit vecteur étant de préférence un fragment d'ADN ou un plasmide.

27. Procédé selon la revendication 26, caractérisé en ce que ledit vecteur contient de l'ADN, qui est homologue à l'ADN dudit micro-organisme, conduisant à l'intégration dans le génome dudit micro-organisme.

28. Procédé de fabrication d'éthanol, caractérisé en ce qu'on utilise un micro-organisme selon l'une quelconque des revendications 17 à 23.

29. Procédé selon la revendication 28, caractérisé en ce que le procédé de fermentation est adapté à la production de boissons alcoolisées ou d'une protéine de cellule unique produite à partir de substrats contenant du xylose libre, de préférence libéré par l'activité de la xylanase et/ou de la xylosidase.

30. Procédé pour la production de biomasse, caractérisé en ce que l'on utilise le micro-organisme hôte selon l'une quelconque des revendications 17 à 23.

## Revendications (Revendications pour l'(les) Etat(s) contractant(s) suivant(s): ES)

1. Procédé de préparation d'une séquence d'ADN, cette séquence d'ADN comprenant un gène de structure codant pour une réductase du xylose ayant la séquence suivante d'acides aminés : 
10
M P S I K L N S G Y
20
D M P A V G F G C W
30
K V D V D T C S E Q
40
I Y R A I K T G Y R
50
L F D G A E D Y A N
60
E K L V G A G V K K
70
A I D E G I V K R E
80
D L F L T S K L W N
90
N Y H H P D N V E K
100
A L N R T L S D L Q
110
V D Y V D L F L I H
120
F P V T F K F V P L
130
E E K Y P P G F Y C
140
G K G D N F D Y E D
 
150
V P I L E T W K A L
160
E K L V K A G K I R
170
S I G V S N F P G A
180
L L L D L L R G A T
190
I K P S V L Q V E H
200
H P Y L Q Q P R L I
210
E F A Q S R G I A V
220
T A Y S S F G P Q S
230
F V E L N Q G R A L
240
N T S P L F E N E T
250
I K A I A A K H G K
260
S P A Q V L L R W S
270
S Q R G I A I I P K
280
S N T V P R L L E N
 
290
K D V N S F D L D E
300
Q D F A D I A K L D
310
I N L R F N D P W D
W D K I P I F V *
 ladite séquence d'ADN étant capable d'exprimer ledit polypeptide dans un micro-organisme, dans lequel ladite séquence d'ADN est préparée par la technologie de l'ADN recombinant, à partir d'ADN naturel et/ou d'ADNc et/ou d'ADN synthétisé chimiquement.

2. Procédé selon la revendication 1, dans lequel ladite séquence d'ADN comprend en outre un gène de structure encodant pour la déshydrogénase du xylitol ayant la séquence suivante d'acides aminés :

10
M T A N P S L V L N
20
K I D D I S F E T Y
30
D A P E I S E P T D
40
V L V Q V K K T G I
50
C G S D I H F Y A H
60
G R I G N F V L T K
70
P M V L G H E S A G
80
T V V Q V G K G V T
90
S L K V G D N V A I
100
E P G I P S R F S D
110
E Y K S G H Y N L C
120
P H M A F A A T P N
130
S K E G E P N P P G

140
T L C K Y F K S P E
150
D F L V K L P D H V
160
S L E L G A L V E P
170
L S V G V H A S K L
180
G S V A F G D Y V A
190
V F G A G P V G L L
200
A A A V A K T F G A
210
K G V I V V D I F D
220
N K L K M A K D I G
230
A A T H T F N S K T
240
G G S E E L I K A F
250
G G N V P N V V L E
260
C T G A E P C I K L

270
G V D A I A P G G R
280
F V Q V G N A A G P
290
V S F P I T V F A M
300
K E L T L F G S F R
310
Y G F N D Y K T A V
320
G I F D T N Y Q N G
330
R E N A P I D F E Q
340
L I T H R Y K F K D
350
A I E A Y D L V R A
360
G K G A V K C L I D
G P E *

3. Procédé selon l'une quelconque des revendications 1 ou 2, caractérisé en ce que ladite séquence d'ADN est dérivée d'une levure, de préférence d'une levure choisie parmi un groupe constitué des genres Schwanniomyces, Saccharomyces, Kluyveromyces, Pichia, Hansenula, Candida, Debaryomyces, Metschnikowia, et Pachysolen.

4. Procédé selon la revendication 3, caractérisé en ce que la levure est Pichia stipitis, de préférence Pichia stipitis 5773 (DSM 5855).

5. Procédé selon la revendication 1, dans lequel la séquence d'ADN comprend la séquence suivante de nucléotides :

-350
GGATCCACAGACACTAATTGGTTCTA
-310
CATTATTCGTGTTCAGACACAAACCCCAGC
-290
GTTGGCGGTTTCTGTCTGCGTTCCTCCAGC
-250
ACCTTCTTGCTCAACCCCAGAAGGTGCACA
-230
CTGCAGACACACATACATACGAGAACCTGG
-190
AACAAATATCGGTGTCGGTGACCGAAATGT
-170
GCAAACCCAGACACGACTAATAAACCTGGC
-130
AGCTCCAATACCGCCGACAACAGGTGAGGT
-110
GACCGATGGGGTGCCAATTAATGTCTGAAA
-70
ATTGGGGTATATAAATATGGCGATTCTCCG
-50
GAGAATTTTTCAGTTTTCTTTTCATTTCTC
-10
CAGTATTCTTTTCTATACAACTATACTACA
10 30
ATGCCTTCTATTAAGTTGAACTCTGGTTAC
50
GACATGCCAGCCGTCGGTTTCGGCTGTTGG

70 90
AAAGTCGACGTCGACACCTGTTCTGAACAG

110
ATCTACCGTGCTATCAAGACCGGTTACAGA

130 150
TTGTTCGACGGTGCCGAAGATTACGCCAAC

170
GAAAAGTTAGTTGGTGCCGGTGTCAAGAAG

190 210
GCCATTGACGAAGGTATCGTCAAGCGTGAA

230
GACTTGTTCCTTACCTCCAAGTTGTGGAAC

250 270
AACTACCACCACCCAGACAACGTCGAAAAG

290
GCCTTGAACAGAACCCTTTCTGACTTGCAA

310 330
GTTGACTACGTTGACTTGTTCTTGATCCAC

350
TTCCCAGTCACCTTCAAGTTCGTTCCATTA

370 390
GAAGAAAAGTACCCACCAGGATTCTACTGT

410
GGTAAGGGTGACAACTTCGACTACGAAGAT

430 450
GTTCCAATTTTAGAGACCTGGAAGGCTCTT

470
GAAAAGTTGGTCAAGGCCGGTAAGATCAGA

490 510
TCTATCGGTGTTTCTAACTTCCCAGGTGCT

530
TTGCTCTTGGACTTGTTGAGAGGTGCTACC

550 570
ATCAAGCCATCTGTCTTGCAAGTTGAACAC

590
CACCCATACTTGCAACAACCAAGATTGATC

610 630
GAATTCGCTCAATCCCGTGGTATTGCTGTC

650
ACCGCTTACTCTTCGTTCGGTCCTCAATCT

670 690
TTCGTTGAATTGAACCAAGGTAGAGCTTTG

710
AACACTTCTCCATTGTTCGAGAACGAAACT

730 750
ATCAAGGCTATCGCTGCTAAGCACGGTAAG

770
TCTCCAGCTCAAGTCTTGTTGAGATGGTCT

790 810
TCCCAAAGAGGCATTGCCATCATTCCAAAG

830
TCCAACACTGTCCCAAGATTGTTGGAAAAC

850 870
AAGGACGTCAACAGCTTCGACTTGGACGAA

890
CAAGATTTCGCTGACATTGCCAAGTTGGAC

910 930
ATCAACTTGAGATTCAACGACCCATGGGAC

950
TGGGACAAGATTCCTATCTTCGTCTAAGAA

970 990
GGTTGCTTTATAGAGAGGAAATAAAACCTA

1010
ATATACATTGATTGTACATTTAAAATTGAA

1030 1050
TATTGTAGCTAGCAGATTCGGAAATTTAAA

1070
ATGGAAGGTGATTCTATCCGTACGAATGA

1090 1110
TCTCTATGTACATACACGTTGAAGATAGCA

1130
GTACAGTAGACATCAAGTCTACAGATCATT

1150 1170
AAACATATCTTAAATTGTAGAAAACTATAA

1190
ACTTTTCAATTCAAACCATGTCTGCCAAGG

1210 1230
AATCAAATGAGATTTTTTCGCAGCCAAAC

1250
TTGAATCCAAAAATAAAAACGTCATTGTC

1270 1290
TGAAACAACTCTATCTTATCTTTCACCTCA

1310
TCAATTCATTGCATATCATAAAAGCCTCCG

1330 1350
ATAGCATACAAAACTACTTCTGCATCATAT

1370
CTAAATCATAGTGCCATATTCAGTAACAAT

1390 1410
ACCGGTAAGAAACTTCTATTTTTTTAGTCT

1430
GCCTTAACGAGATGCAGATCGATGCAACGT

1450 1470
AAGATCAAACCCCTCCAGTTGTACAGTCAG

1490
TCATATAGTGAACACCGTACAATATGGTAT

1510 1530
CTACGTTCAAATAGACTCCAATACAGCTGG

1550
TCTGCCCAAGTTGAGCAACTTTAATTTAGA

1570 1590
GACAAAGTCGTCTCTGTTGATGTAGGCACC

1610
ACACATTCTTCTCTTGCCCGTGAACTCTGT

1630 1650
TCTGGAGTGGAAACATCTCCAGTTGTCAAA

1670
TATCAAACACTGACCAGGCTTCAACTGGTA

1690
GAAGATTTCGTTTTCGGGATC

6. Procédé selon la revendication 2, dans lequel la séquence d'ADN comprend la séquence suivante de nucléotides :

-310 -290
TCTAGACCACCCTAAGTCGTCCCTATGTCG

-270
TATGTTTGCCTCTACTACAAAGTTACTAGC

-250 -230
AAATATCCGCAGCAACAACAGCTGCCCTCT

-210
TCCAGCTTCTTAGTGTGTTGGCCGAAAAGG

-190 -170
CGCTTTCGGGCTCCAGCTTCTGTCCTCTGC

-150
GGCTGCTGCACATAACGCGGGACAATGAC

-130 -110
TTCTCCAGCTTTTATTATAAAAGGAGCCAT

-90
CTCCTCCAGGTGAAAAATTACGATCAACTT

-70 -50
TTACTCTTTTCCATTGTCTCTTGTGTATAC

-30
TCACTTTAGTTTGTTTCAATCACCCCTAAT

-10 10
ACTCTTCACACAATTAAAATGACTGCTAAC

30
CCTTCCTTGGTGTTGAACAAGATCGACGAC

50 70
ATTTCGTTCGAAACTTACGATGCCCCAGAA

90
ATCTCTGAACCTACCGATGTCCTCGTCCAG

110 130
GTCAAGAAAACCGGTATCTGTGGTTCCGAC

150
ATCCACTTCTACGCCCATGGTAGAATCGGT

170 190
AACTTCGTTTTGACCAAGCCAATGGTCTTG

210
GGTCACGAATCCGCCGGTACTGTTGTCCAG

230 250
GTTGGTAAGGGTGTCACCTCTCTTAAGGTT

270
GGTGACAACGTCGCTATCGAACCAGGTATT

290 310
CCATCCAGATTCTCCGACGAATACAAGAGC

330
GGTCACTACAACTTGTGTCCTCACATGGCC

350 370
TTCGCCGCTACTCCTAACTCCAAGGAAGGC

390
GAACCAAACCCACCAGGTACCTTATGTAAG

410 430
TACTTCAAGTCGCCAGAAGACTTCTTGGTC

450
AAGTTGCCAGACCACGTCAGCTTGGAACTC

470 490
GGTGCTCTTGTTGAGCCATTGTCTGTTGGT

510
GTCCACGCCTCCAAGTTGGGTTCCGTTGCT

530 550
TTCGGCGACTACGTTGCCGTCTTTGGTGCT

570
GGTCCTGTTGGTCTTTTGGCTGCTGCTGTC

590 610
GCCAAGACCTTCGGTGCTAAGGGTGTCATC

630
GTCGTTGACATTTTCGACAACAAGTTGAAG

650 670
ATGGCCAAGGACATTGGTGCTGCTACTCAC

690
ACCTTCAACTCCAAGACCGGTGGTTCTGAA

710 730
GAATTGATCAAGGCTTTCGGTGGTAACGTG

750
CCAAACGTCGTTTTGGAATGTACTGGTGCT

770 790
GAACCTTGTATCAAGTTGGGTGTTGACGCC

810
ATTGCCCCAGGTGGTCGTTTCGTTCAAGTT

830 850
GGTAACGCTGCTGGTCCAGTCAGCTTCCCA

870
ATCACCGTTTTCGCCATGAAGGAATTGACT

890 910
TTGTTCGGTTCTTTCAGATACGGATTCAAC

930
GACTACAAGACTGCTGTTGGAATCTTTGAC

950 970
ACTAACTACCAAAACGGTAGAGAAAATGCT

990
CCAATTGACTTTGAACAATTGATCACCCAC

1010 1030
AGATACAAGTTCAAGGACGCTATTGAAGCC

1050
TACGACTTGGTCAGAGCCGGTAAGGGTGCT

1070 1090
GTCAAGTGTCTCATTGACGGCCCTGAGTAA

1110
GTCAACCGCTTGGCTGGCCCAAAGTGAACC

1130 1150
AGAAACGAAAATGATTATCAAATAGCTTTA

1170
TAGACCTTTATCGAAATTTATGTAAACTAA

1190 1210
TAGAAAAGACAGTGTAGAAGTTATATGGTT

1230
GCATCACGTGAGTTTCTTGAATTCTTGAAA

1250                                    1270
GTGAAGTCTTGGTCGGAACAAACAAACAAA

                    1290
AAAATATTTTCAGCAAGAGTTGATTTCTTT

1310                                    1330
TCTGGAGATTTTGGTAATTGACAGAGAACC

                    1350
CCTTTCTGCTATTGCCATCTAAACATCCTT

1370                                    1390
GAATAGAACTTTACTGGATGGCCGCCTAGT

                    1410
GTTGAGTATATATTATCAACCAAAATCCTG

1430                                    1450
TATATAGTCTCTGAAAAATTTGACTATCCT

                    1470
AACTTAACAAAAGAGCACCATAATGCAAGC

1490                                    1510
TCATAGTTCTTAGAGACACCAACTATACTT

                    1530
AGCCAAACAAAATGTCCTTGGCCTCTAAAG

1550                                    1570
AAGCATTCAGCAAGCTTCCCCAGAAGTTGC

                    1590
ACAACTTCTTCATCAAGTTTACCCCCAGAC

1610                                    1630
CGTTTGCCGAATATTCGGAAAAGCCTTCGA

CTATAGTGGATCC

7. Procédé de préparation d'une combinaison de séquences d'ADN, ledit procédé comprenant la combinaison d'une première séquence d'ADN susceptible d'être obtenue selon l'une quelconque des revendications 1 à 6 et une ou plusieurs autres séquences d'ADN capables de réguler l'expression d'un gène de structure encodé par ladite séquence d'ADN dans un micro-organisme hôte d'une manière connue en soi.

8. Procédé selon la revendication 7, dans lequel ladite combinaison de séquences comprend des modifications des séquences d'ADN conservant leur capacité à exprimer une enzyme fonctionnelle ayant une activité de réductase du xylose ou de déshydrogénase du xylitol.

9. Procédé selon l'une quelconque des revendications 7 ou 8, dans lequel ledit gène de structure contient des séquences d'ADN dérivées du gène de structure codant pour la réductase du xylose ou la déshydrogénase du xylitol qui modifie ledit produit de protéine tout en conservant ses fonctions d'une façon telle que ledit produit de protéine est exprimé comme un produit de gène ayant une activité enzymatique.

10. Procédé selon l'une quelconque des revendications 7 à 9, dans lequel lesdites séquences d'ADN capables de réguler l'expression dudit gène de structure dans un micro-organisme hôte, sont dérivées dudit micro-organisme hôte.

11. Procédé selon la revendication 10, dans lequel lesdites séquences d'ADN capables de réguler l'expression sont des promoteurs susceptibles d'être induits.

12. Procédé selon la revendication 11, caractérisé en ce que lesdites séquences d'ADN capables de réguler l'expression sont choisies parmi les promoteurs suivants :
ADH1, ADH2, PDC, GAL1/10.

13. Procédé selon l'une quelconque des revendications 10 à 12, dans lequel ladite séquence d'ADN capable de réguler l'expression dudit gène de structure, est un promoteur fort, conduisant à une surexpression de la protéine encodée par ledit gène de structure.

14. Procédé de préparation d'un vecteur, ledit procédé comprenant l'insertion d'une séquence d'ADN susceptible d'être obtenue selon l'une quelconque des revendications 1 à 6 ou d'une combinaison de séquences d'ADN susceptibles d'être obtenues selon l'une quelconque des revendications 7 à 13, dans un plasmide hôte.

15. Procédé selon la revendication 14, caractérisé en ce qu'il produit un vecteur choisi parmi le groupe comprenant les plasmides pR1, pR2, pD1, pD2, pRD1, pXRa, pXRb, pXDH, pXR, pXDH-HIS3, pXR-LEU2.

16. Procédé de préparation d'un micro-organisme capable d'exprimer une réductase du xylose ou une réductase du xylose et une deshydrogènase du xylitol, dans lequel des séquences d'ADN comprenant les séquences d'ADN susceptibles d'être obtenues selon l'une quelconque des revendications 1 à 6 ou une combinaison de séquences d'ADN susceptibles d'être obtenues selon l'une quelconque des revendications 7 à 13, codant pour ladite réductase du xylose ou ladite réductase du xylose et ladite deshydrogènase du xylitol, sont introduites dans un micro-organisme hôte.

17. Procédé selon la revendication 16, caractérisé en ce que ledit micro-organisme hôte est choisi parmi un groupe constitué de levures des genres Saccharomyces, Schizosaccharomyces, Schwanniomyces, Kluyveromyces, Pichia, Hansenula, Candida, Debaryomyces, Metschnikowia, Pachysolen ou Paecilomyces ou de bactéries du genre Zymomonas.

18. Procédé selon la revendication 17, caractérisé en ce que ledit micro-organisme est Saccharomyces cerevisiae.

19. Procédé selon la revendication 17, caractérisé en ce que ledit micro-organisme est Schizosaccharomyces pombe.

20. Procédé selon l'une quelconque des revendications 16 à 19, caractérisé en ce que ladite séquence d'ADN ou la combinaison de séquences d'ADN est intégrée dans le génome dudit micro-organisme.

21. Procédé selon l'une quelconque des revendications 17 à 21, caractérisé en ce que ledit micro-organisme est utile dans la production de biomasse, dans l'industrie alimentaire et dans les procédés de fermentation.

22. Procédé selon la revendication 21, caractérisé en ce que ledit micro-organisme est utile pour la fermentation du xylose dans l'éthanol.

23. Procédé de production de la réductase du xylose ou de la réductase du xylose et de la déshydrogénase du xylitol par culture d'un micro-organisme susceptible d'être obtenu selon l'une quelconque des revendications 16 à 20 sous des conditions appropriées et récupération du(des)dits enzyme(s) d'une manière connue en soi.

24. Procédé selon la revendication 23, caractérisé en ce que ledit micro-organisme est choisi pour la fermentation efficace du xylulose.

25. Procédé selon la revendication 23 ou 24, caractérisé en ce que ledit micro-organisme a reçu lesdites séquences d'ADN ou ladite combinaison de séquences d'ADN par transformation utilisant un vecteur, ledit vecteur étant de préférence un fragment d'ADN ou un plasmide.

26. Procédé selon la revendication 25, caractérisé en ce que ledit vecteur contient de l'ADN, qui est homologue à l'ADN dudit micro-organisme, conduisant à l'intégration dans le génome dudit micro-organisme.

27. Procédé de fabrication d'éthanol, caractérisé en ce qu'on utilise un micro-organisme selon l'une quelconque des revendications 16 à 22.

28. Procédé selon la revendication 27, caractérisé en ce que le procédé de fermentation est adapté à la production de boissons alcoolisées ou d'une protéine de cellule unique produite à partir de substrats contenant du xylose libre, de préférence libéré par l'activité de la xylanase et/ou de la xylosidase.

29. Procédé pour la production de biomasse, caractérisé en ce que l'on utilise le micro-organisme hôte selon l'une quelconque des revendications 16 à 22.
